# EUROPEAN PATENT APPLICATION

(11) **EP 3 168 234 A1**
(43) Date of publication of application: **17.05.2017**
(21) Application number: 16199226.8
(22) Date of filing: 12.12.2006
(51) Int. Cl.: C07K 16/22, C07K 16/28, A61P 35/00, A61K 39/395

(54) **COMBINATION OF ANGIOPOIETIN-2 ANTAGONIST AND OF VEGF-A, KDR AND/OR FLTL ANTAGONIST FOR TREATING CANCER**

(30) Priority: 15.12.2005 US 750551 P
(62) Divisional of application: 11167374.5
(71) Applicant: MEDIMMUNE LIMITED, Cambridge Cambridgeshire CB21 6GH (GB)
(72) Inventor: BROWN, Jeffrey Lester, Boston, MA 02451 (US); EMERY, Stephen Charles, Macclesfield, Cheshire SK10 4TG (GB); BLAKEY, David Charles, Macclesfield, Cheshire SK10 4TG (GB)
(74) Representative: Winter, Christopher Spencer

(57) **Abstract**

The invention relates to agents which possess anti-angiogenic activity and are accordingly useful in methods of treatment of disease states associated with angiogenesis in the animal or human body. More specifically the invention concerns a combination of an antagonist of the biological activity of Angiopoietin-2 and an antagonist of the biological activity of VEGF-A, and/or KDR, and/or Flt1, and uses of such antagonists.

SEQUENCE LISTING

<110>  AstraZeneca AB

<120>  COMBINATIONS

<130>  CCH 102111

<160>  40

<170>  PatentIn version 3.2

<210>  1
<211>  366
<212>  DNA
<213>  Homo sapiens

<400>  1
gaggtgcagc tggtggagtc tgggggaggc ttggtccagc ctggggggtc cctgagactc      60

tcctgtgcag tctctggatt cacctttagt agctattgga tgagctgggt ccgccaggct     120

ccagggaagg ggctggagtg ggtggccaac ataaagcaag atggaagtga gaaatactat     180

gtggactctg tgaagggccg attcaccatc tccagagaca acgccaagaa ctcactgtat     240

ctgcaaatga acagcctgag agccgaggac acggctgtgt attactgtgc gagagatcaa     300

ggtatagcag tggctgggcc ctttgactac tggggccagg gaaccctggt caccgtctcc     360

tcagcc                                                                366

<210>  2
<211>  122
<212>  PRT
<213>  Homo Sapiens

<400>  2

Glu Val Gln Leu Val Glu Ser Gly Gly Gly Leu Val Gln Pro Gly Gly
1               5                   10                  15

Ser Leu Arg Leu Ser Cys Ala Val Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Gln Gly Ile Ala Val Ala Gly Pro Phe Asp Tyr Trp Gly
            100                 105                 110

Gln Gly Thr Leu Val Thr Val Ser Ser Ala
        115                 120

<210>  3
<211>  322
<212>  DNA
<213>  Homo sapiens

<400>  3
gaaatagtga tgacgcagtc tccagccacc ctgtctgtgt ctccagggga aagagccacc      60

ctctcctgca gggccagtca gactgttagc agcgacttag cctggtacca gcagaaacct     120

ggccaggctc ccaggctcct catctatgga gcatccatta gggccactgg tatcccagcc     180

aggttcagtg gcagtgggtc tgggacagag ttcactctca ccatcagcag cctgcagtct     240

gaagattttg cagtttattc ctgtcagcag tattataact ggtggacgtt cggccaaggg     300

accaaggtgg aaatcaaacg aa                                              322

<210>  4
<211>  107
<212>  PRT
<213>  Homo Sapiens

<400>  4

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Thr Val Ser Ser Asp
            20                  25                  30

Leu Ala Trp Tyr Gln Gln Lys Pro Gly Gln Ala Pro Arg Leu Leu Ile
        35                  40                  45

Tyr Gly Ala Ser Ile Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Ser Cys Gln Gln Tyr Tyr Asn Trp Trp Thr
                85                  90                  95

Phe Gly Gln Gly Thr Lys Val Glu Ile Lys Arg
            100                 105

<210>  5
<211>  372
<212>  DNA
<213>  Homo sapiens

<400>  5
caggtgcagc tggtggagtc tgggggaggc gtggtccagc ctgggaggtc cctgagactc      60

tcctgtgcag cctctggatt caccttcact aactatggca tgcactgggg ccgccaggct     120

ccaggcaagg ggctggagtg ggtggcagtt atatcacatg atggaaataa taagtattat     180

gtagactccg tgaagggccg attcaccatc tccagagaca attccaagaa cacgctgtat     240

ctgcaaatga acagcctgag agctgaggac acggctgtgt attactgtgc gagagaggga     300

atcgattttt ggagtggcct caactggttc gacccctggg gccagggaac cctggtcacc     360

gtctcctcag cc                                                         372


<210>  6
<211>  124
<212>  PRT
<213>  Homo Sapiens

<400>  6

Gln Val Gln Leu Val Glu Ser Gly Gly Gly Val Val Gln Pro Gly Arg
1               5                   10                  15


Ser Leu Arg Leu Ser Cys Ala Ala Ser Gly Phe Thr Phe Thr Asn Tyr
            20                  25                  30


Gly Met His Trp Gly Arg Gln Ala Pro Gly Lys Gly Leu Glu Trp Val
        35                  40                  45


Ala Val Ile Ser His Asp Gly Asn Asn Lys Tyr Tyr Val Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Glu Gly Ile Asp Phe Trp Ser Gly Leu Asn Trp Phe Asp Pro
            100                 105                 110


Trp Gly Gln Gly Thr Leu Val Thr Val Ser Ser Ala
        115                 120


<210>  7
<211>  327
<212>  DNA

<213>  Homo Sapiens

<400>  7
gaaattgtgt tgacgcagtc tccaggcacc ctgtctttgt ctccagggga aagagccact      60

ctctcctgca gggccagtca gagtattacc ggcagctact tagcctggta ccagcagaaa     120

cctggccagg ctcccagact cctcatctgt ggtgcatcca gctgggccac tggcatccca     180

gacaggttca gtggcagtgg gtctgggaca gacttcactc tcaccatcag tagactggag     240

cctgaagatt ttgcagtgta ttactgtcag cagtatagta gttcaccgat caccttcggc     300

caagggacac gactggagat taaacga                                         327


<210>  8
<211>  109
<212>  PRT
<213>  Homo Sapiens

<400>  8

Glu Ile Val Leu Thr Gln Ser Pro Gly Thr Leu Ser Leu Ser Pro Gly
1               5                   10                  15


Glu Arg Ala Thr Leu Ser Cys Arg Ala Ser Gln Ser Ile Thr Gly Ser
            20                  25                  30


Tyr Leu Ala Trp Tyr Gln Gln Lys Pro Gly Gln Ala Pro Arg Leu Leu
        35                  40                  45


Ile Cys Gly Ala Ser Ser Trp Ala Thr Gly Ile Pro Asp Arg Phe Ser
    50                  55                  60


Gly Ser Gly Ser Gly Thr Asp Phe Thr Leu Thr Ile Ser Arg Leu Glu
65                  70                  75                  80


Pro Glu Asp Phe Ala Val Tyr Tyr Cys Gln Gln Tyr Ser Ser Ser Pro
                85                  90                  95


Ile Thr Phe Gly Gln Gly Thr Arg Leu Glu Ile Lys Arg
            100                 105


<210>  9
<211>  357
<212>  DNA
<213>  Homo sapiens

<400>  9
gaggtgcagc tggtggagtc tgggggaggc ttggtccagc ctggggggtc cctgagactc      60

tcctgtgcag cctctggatt cacctttagt agctattgga tgagctgggt ccgccaggct     120

ccagggaagg ggctggagtg ggtggccaac ataaagcaag atggaagtga caaatactat     180

gtggactctg tgaagggccg attcaccatc tccagagaca acgccaagaa ctcactgtat 240

ctgcgaatga acagcctgag agccgaggac acggctgtgt tttactgtgc gagagatatg 300

ggcagtggct ggtttgacta ctggggccag ggaaccctgg tcaccgtctc ctcagcc 357

<210> 10
<211> 119
<212> PRT
<213> Homo sapiens

<400> 10

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Lys Gln Asp Gly Ser Asp Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Arg Met Asn Ser Leu Arg Ala Glu Asp Thr Ala Val Phe Tyr Cys
                85                  90                  95

Ala Arg Asp Met Gly Ser Gly Trp Phe Asp Tyr Trp Gly Gln Gly Thr
            100                 105                 110

Leu Val Thr Val Ser Ser Ala
        115

<210> 11
<211> 321
<212> DNA
<213> Homo Sapiens

<400> 11
gaagtagtga tgacgcagtc tccagccacc ctgtctgtgt ctccagggga aagagccacc 60

ctctcctgca gggccagtca gagtgttggc agcaacttag cctggtacca gcagaaacct 120

ggccaggctc ccaggctcct catctatggt gcatccacca gggccactgg tatcccagcc 180

aggttcagtg gcagtgggtc tgggacagag ttcactctca ccatcagcag cctgcagtct 240

gaagattttg cagtttattg ctgtcagcag tataatcact ggtggacgtt cggccaaggg 300

accaaggtgg aaatcaaacg a 321

<210>  12
<211>  107
<212>  PRT
<213>  Homo sapiens

<400>  12

Glu Val Val Met Thr Gln Ser Pro Ala Thr Leu Ser Val Ser Pro Gly
1               5                   10                  15

Glu Arg Ala Thr Leu Ser Cys Arg Ala Ser Gln Ser Val Gly Ser Asn
            20                  25                  30

Leu Ala Trp Tyr Gln Gln Lys Pro Gly Gln Ala Pro Arg Leu Leu Ile
        35                  40                  45

Tyr Gly Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Cys Cys Gln Gln Tyr Asn His Trp Trp Thr
                85                  90                  95

Phe Gly Gln Gly Thr Lys Val Glu Ile Lys Arg
            100                 105


<210>  13
<211>  365
<212>  DNA
<213>  Homo Sapiens

<400>  13
caggtacagc tggtgcagtc tggggctgag gtgaagaagc ctggggcctc agtgaaggtc      60

tcctgcaagg cttctggata caccttcacc ggcttctata tgtactgggt gcgacaggcc     120

cctggacaag ggcttgagtg gatgggatgg atcaacccta acagtagtgg cacaaaccat     180

gcacagaagt ttcagggcag ggtcaccatg accagggaca cgtccatcag cacagcctac     240

atggagctga gcaggctgag atctgacgac acggccgtgt attactgtgc gagagatcag     300

gatatagcaa cagctggtcc ctttgactac tggggccagg gaaccctggt caccgtctcc     360

tcagc                                                                 365


<210>  14
<211>  121
<212>  PRT
<213>  Homo sapiens

<400>  14

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Phe
            20                  25                  30

Tyr Met Tyr Trp Val Arg Gln Ala Pro Gly Gln Gly Leu Glu Trp Met
        35                  40                  45

Gly Trp Ile Asn Pro Asn Ser Ser Gly Thr Asn His Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Asp Gln Asp Ile Ala Thr Ala Gly Pro Phe Asp Tyr Trp Gly
            100                 105                 110

Gln Gly Thr Leu Val Thr Val Ser Ser
        115                 120

<210>  15
<211>  322
<212>  DNA
<213>  Homo sapiens

<400>  15
gaaatagtga tgacgcagtc tccagccacc ctgtctgtgt ctccagggga aagagccacc      60

ctctcctgca gggccagtca gagtgttagc agcaacttag cctggtacca gcagaaacct     120

ggccaggctc ccaggctcct catctttggt gcatccaccc gggccactgg tatcccagcc     180

aggttcagtg gcagtgggtc tgggacagag ttcactctca ccatcagcag cctgcagtct     240

gaagattttg cagtttatta ctgtcagcag tataataact ggtggacgtt cggccgaggg     300

accaaggtgg aaatcaaacg aa                                              322

<210>  16
<211>  107
<212>  PRT
<213>  Homo Sapiens

<400>  16

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Asn
            20                  25                  30

Leu Ala Trp Tyr Gln Gln Lys Pro Gly Gln Ala Pro Arg Leu Leu Ile
        35                  40                  45

Phe Gly Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Asn Trp Trp Thr
                85                  90                  95

Phe Gly Arg Gly Thr Lys Val Glu Ile Lys Arg
            100                 105

<210>  17
<211>  369
<212>  DNA
<213>  Homo Sapiens

<400>  17
gaagtgcagc tggtggagtc tgggggaatc gtggtacagc ctggggggtc cctgagactc      60

tcctgtgcag cctctggatt cacctttgat gattatacca tgcactgggt ccgtcaaact     120

ccggggaagg gtctggagtg ggtctctctt attagttggg atggtggtag cacatactat     180

gcagactctg tgaagggccg attcaccatc tccagagaca acagcaaaaa ctccctgtat     240

ctgcaaatga acagtctgag aactgaggac accgccttgt attactgtgc aaaagatata     300

gatatagcag tggctggtac aggatttgac cactggggcc agggaaccct ggtcaccgtc     360

tcctcagct                                                             369

<210>  18
<211>  123
<212>  PRT
<213>  Homo sapiens

<400>  18

Glu Val Gln Leu Val Glu Ser Gly Gly Ile Val Val Gln Pro Gly Gly
1               5                   10                  15

Ser Leu Arg Leu Ser Cys Ala Ala Ser Gly Phe Thr Phe Asp Asp Tyr
            20                  25                  30

Thr Met His Trp Val Arg Gln Thr Pro Gly Lys Gly Leu Glu Trp Val
        35                  40                  45

Ser Leu Ile Ser Trp Asp Gly Gly Ser Thr Tyr Tyr Ala Asp Ser Val
    50                  55                  60

Lys Gly Arg Phe Thr Ile Ser Arg Asp Asn Ser Lys Asn Ser Leu Tyr
65                  70                  75                  80

Leu Gln Met Asn Ser Leu Arg Thr Glu Asp Thr Ala Leu Tyr Tyr Cys
                85                  90                  95

Ala Lys Asp Ile Asp Ile Ala Val Ala Gly Thr Gly Phe Asp His Trp
            100                 105                 110

Gly Gln Gly Thr Leu Val Thr Val Ser Ser Ala
        115                 120

<210>  19
<211>  324
<212>  DNA
<213>  Homo Sapiens

<400>  19
gaaatagtga tgacgcagtc tccagccacc ctgtctgtgt ctccagggga aagagccacc      60

ctctcctgca gggccagtca gagtgttacc agcaacctag cctggtacca gcagaaacct     120

ggccaggctc ccaggctcct catctatggt gcattaatta gggccactgg tatcccagcc     180

aggttcagtg gcagtgggtc tgggacagag ttcactctca ccatcagcag cctgcagtct     240

gaagattttg cagtttatta ctgtcagcaa tataataact ggccattcac tttcggccct     300

gggaccaaag tggatatcaa acga                                            324

<210>  20
<211>  108
<212>  PRT
<213>  Homo Sapiens

<400>  20

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Thr Ser Asn
            20                  25                  30

Leu Ala Trp Tyr Gln Gln Lys Pro Gly Gln Ala Pro Arg Leu Leu Ile
        35                  40                  45

Tyr Gly Ala Leu Ile Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Asn Trp Pro Phe
                85                  90                  95

Thr Phe Gly Pro Gly Thr Lys Val Asp Ile Lys Arg
            100                 105

<210>  21
<211>  378
<212>  DNA
<213>  Homo Sapiens

<400>  21
caggtgcagc tggtgcagtc tggggctgag gtgaagaagc ctggggcctc agtgaaggtc      60

tcctgcaagg cttctggata caccttcacc ggctactata tgcactgggt gcgacaggcc     120

cctggacaag ggcttgagtg gatgggatgg atcaacccta acagtggtgg cacaaactat     180

gcacagaagt ttcagggcag ggtcaccatg accagggaca cgtccatcag cacagcctac     240

atggagctga gcaggctgag atctgacgac acggccgtgt attactgtgc gagagatagg     300

ggctggaact acgcagacta ctactactac ggtatggacg tctggggcca agggaccacg     360

gtcaccgtct cctcagct                                                   378

<210>  22
<211>  126
<212>  PRT
<213>  Homo sapiens

<400>  22

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Asp Arg Gly Trp Asn Tyr Ala Asp Tyr Tyr Tyr Tyr Gly Met
            100                 105                 110

Asp Val Trp Gly Gln Gly Thr Thr Val Thr Val Ser Ser Ala
        115                 120                 125

<210>  23
<211>  342
<212>  DNA
<213>  Homo sapiens

<400>  23
gacatcgtga tgacccagtc tccagactcc ctggctgtgt ctctgggcga gagggccacc      60

atcaactgca agtccagcca gagtgtttta tacagttcca acaatcagaa cttcttagct     120

tggtatcagc agaaaccagg acagcctcct aaactgctca tttactgggc atctacccgg     180

gaatccgggg tccctgaccg attcagtggc agcgggtctg ggacagattt cactctcacc     240

atcagcagcc tgcaggctga agatgtggca gtttattact gtcaccaata ttatagtact     300

ccgatcacct tcggccaagg gacacgactg gagattaaac ga                        342

<210>  24
<211>  114
<212>  PRT
<213>  Homo Sapiens

<400>  24

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Val Leu Tyr Ser
            20                  25                  30

Ser Asn Asn Gln Asn Phe Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys His Gln
                85                  90                  95

Tyr Tyr Ser Thr Pro Ile Thr Phe Gly Gln Gly Thr Arg Leu Glu Ile
            100                 105                 110

Lys Arg

<210> 25
<211> 366
<212> DNA
<213> Homo Sapiens

<400> 25
caggtgcagc tggtgcagtc cggggctgag gtgaagaagc ctggggcctc agtgaaggtc 60

tcctgcaagg cttctggata caccttcacc ggctaccata tgtactgggt gcgacaggcc 120

cctggacaag ggcttgagtg gctgggatgg atcaacccta acagtggtgg cacaaactat 180

gcacagaagt ttcagggcag ggtcaccatg accagggaca cgtccatcag cacagcctac 240

atggagctga gcaggctgag atctgacgac acggccgtgt attactgtgt gagagatcag 300

ggtatagcag cagctggtcc ctttgactac tggtgccagg gaaccctggt caccgtctcc 360

tcagct 366

<210> 26
<211> 122
<212> PRT
<213> Homo Sapiens

<400> 26

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

His Met Tyr Trp Val Arg Gln Ala Pro Gly Gln Gly Leu Glu Trp Leu
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Val Arg Asp Gln Gly Ile Ala Ala Ala Gly Pro Phe Asp Tyr Trp Cys
            100                 105                 110

Gln Gly Thr Leu Val Thr Val Ser Ser Ala
        115                 120

<210>  27
<211>  324
<212>  DNA
<213>  Homo sapiens

<400>  27
gacatccgga tgacccagtc tccatcctcc ctgtctgcat ctgttggaga cagagtcacc      60

atcacttgcc gggcaagtca gcgcattagc acctatttaa attggtatca gcagaaacca     120

gggaaagccc ctaagttcct gatctatgct gcatctagtt tgcaaagtgg ggtcccatca     180

aggttcagtg gcagtggatc tgggacagat ttcactctca ccatcagcag tctgcaacct     240

gaagattttg caacttacta ctgtcaacag agttacacta ccccattcac tttcggccct     300

gggaccaaag tggatatcaa acga                                            324


<210>  28
<211>  108
<212>  PRT
<213>  Homo sapiens

<400>  28

Asp Ile Arg Met Thr Gln Ser Pro Ser Ser Leu Ser Ala Ser Val Gly
1               5                   10                  15


Asp Arg Val Thr Ile Thr Cys Arg Ala Ser Gln Arg Ile Ser Thr Tyr
            20                  25                  30


Leu Asn Trp Tyr Gln Gln Lys Pro Gly Lys Ala Pro Lys Phe Leu Ile
        35                  40                  45


Tyr Ala Ala Ser Ser Leu Gln Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Ser Tyr Thr Thr Pro Phe
                85                  90                  95


Thr Phe Gly Pro Gly Thr Lys Val Asp Ile Lys Arg
            100                 105


<210>  29
<211>  363
<212>  DNA
<213>  Homo Sapiens

<400>  29
gaggtgcaga tggtggagtc tgggggaggc ttggtccagc cgggggggtc cctgagactc      60

tcctgtgcag cctctggatt caccttaaga agctactgga tgagctgggt ccgccaggct 120

ccagggaagg ggctggagtg ggtggccaac ataaaggaag acggaagtga gaaataccat 180

gtggactctg tgaagggccg attcaccatc tccagagaca acgccgagaa ctcactgttt 240

ctgcaaatga gcagcctgcg agccgaggac acggctgtgt attactgtgc gagagatatg 300

gaagcatcag ctggcctctt tgactactgg ggccagggaa ccctggtcac cgtctcctca 360

gct 363

<210> 30
<211> 121
<212> PRT
<213> Homo Sapiens

<400> 30

Glu Val Gln Met Val Glu Ser Gly Gly Gly Leu Val Gln Pro Gly Gly
1               5                   10                  15

Ser Leu Arg Leu Ser Cys Ala Ala Ser Gly Phe Thr Leu Arg Ser Tyr
            20                  25                  30

Trp Met Ser Trp Val Arg Gln Ala Pro Gly Lys Gly Leu Glu Trp Val
        35                  40                  45

Ala Asn Ile Lys Glu Asp Gly Ser Glu Lys Tyr His Val Asp Ser Val
    50                  55                  60

Lys Gly Arg Phe Thr Ile Ser Arg Asp Asn Ala Glu Asn Ser Leu Phe
65                  70                  75                  80

Leu Gln Met Ser Ser Leu Arg Ala Glu Asp Thr Ala Val Tyr Tyr Cys
                85                  90                  95

Ala Arg Asp Met Glu Ala Ser Ala Gly Leu Phe Asp Tyr Trp Gly Gln
            100                 105                 110

Gly Thr Leu Val Thr Val Ser Ser Ala
        115                 120

<210> 31
<211> 321
<212> DNA
<213> Homo sapiens

<400> 31
gaaatagtga tgacgcagtc cccagccacc ctgtctgtgt ctccagggga aagagccatc 60

ctctcctgca gggccagtca gagtattagc agcaacttag cctggtacca gcagaaacct 120

ggccaggctc ccaggctcct catctatggt gcatccacca gggccactgg tatcccagcc 180

aggttcagtg gcagtgggtc tgggacagag ttcactctca ccatcagcag cctgcagtct 240

gaagattttg cagtttatta ctgtcagcag tataattact ggtggacgtt cggccaaggg 300

accaaggtgg aaatcaaacg a 321

<210> 32
<211> 107
<212> PRT
<213> Homo Sapiens

<400> 32

Glu Ile Val Met Thr Gln Ser Pro Ala Thr Leu Ser Val Ser Pro Gly
1               5                   10                  15

Glu Arg Ala Ile Leu Ser Cys Arg Ala Ser Gln Ser Ile Ser Ser Asn
            20                  25                  30

Leu Ala Trp Tyr Gln Gln Lys Pro Gly Gln Ala Pro Arg Leu Leu Ile
        35                  40                  45

Tyr Gly Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Tyr Trp Trp Thr
                85                  90                  95

Phe Gly Gln Gly Thr Lys Val Glu Ile Lys Arg
            100                 105

<210> 33
<211> 369
<212> DNA
<213> Homo sapiens

<400> 33
caggtgcagc tggtgcagtc tggggctgag gtgaagaagc ctggggcctc agtgaaggtc 60

tcctgcaagg cttctggata caccttcacc ggctactata tgcactgggt gccacaggcc 120

cctggacaag ggcttgagtg gatgggatgg atcaacccta acagtggtgg cacaaactat 180

gcacagaagt ttcagggcag ggtcaccatg accagggaca cgtccatcag cacagcttac 240

atggagctga ggaggctgag atctgacgac acggccgtgt attactgtgc gagagatggg 300

ggtagtatac cagtgtctgg tcactttgac tactggggcc agggaaccct ggtcaccgtc 360

tcctcagct 369

<210>  34
<211>  123
<212>  PRT
<213>  Homo Sapiens

<400>  34

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Pro Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Arg Arg Leu Arg Ser Asp Asp Thr Ala Val Tyr Tyr Cys
                85                  90                  95

Ala Arg Asp Gly Gly Ser Ile Pro Val Ser Gly His Phe Asp Tyr Trp
            100                 105                 110

Gly Gln Gly Thr Leu Val Thr Val Ser Ser Ala
        115                 120


<210>  35
<211>  321
<212>  DNA
<213>  Homo Sapiens

<400>  35
gaaatagtga tgacgcagtc tccagccacc ctgtctgtgt ctccagggga aagagccacc      60

ctctcctgca gggccagtca gagtcttatc agcaacttag cctggtacca gcagaaacct     120

ggccaggctc ccaggctcct catctttggt gcatccacca gggccactgg tatcccagcc     180

aggttcagtg gcagtgggtc tgggacagag ttcactctca ccatcagcag cctgcagtct     240

gaagattttg cagtttatta ctgtcatcag tataataact ggtggacgtt cggccaaggg     300

accaaggtgg aaatcaaacg a                                               321


<210>  36
<211>  107
<212>  PRT

<213>  Homo sapiens

<400>  36

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Leu Ile Ser Asn
            20                  25                  30

Leu Ala Trp Tyr Gln Gln Lys Pro Gly Gln Ala Pro Arg Leu Leu Ile
        35                  40                  45

Phe Gly Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys His Gln Tyr Asn Asn Trp Trp Thr
                85                  90                  95

Phe Gly Gln Gly Thr Lys Val Glu Ile Lys Arg
            100                 105

<210>  37
<211>  366
<212>  DNA
<213>  Homo Sapiens

<400>  37
caggtgcagc tggtgcagtc tggggctgag gtgaaaaagc ctggggcctc agtcaaggtc      60

tcctgcaagg cttctggata caccttcacc ggctactatt tgtactgggt gccacaggcc     120

cctggacaag ggcttgagtg gatgggatgg atcagcccta acagtggtgg cacaaactat     180

gcacagaagt ttcagggcag ggtcaccatg accagggaca cgtccatcag cacagcctac     240

atggagctga gcaggctgag atctgacgac acggccgtgt attactgtgc gagagatcag     300

gtcatagcag tagctggtcc ctttgactac tggggccaag gaaccctggt caccgtctcc     360

tcagct                                                                366

<210>  38
<211>  122
<212>  PRT
<213>  Homo Sapiens

<400>  38

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Leu Tyr Trp Val Pro Gln Ala Pro Gly Gln Gly Leu Glu Trp Met
        35                  40                  45

Gly Trp Ile Ser Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Asp Gln Val Ile Ala Val Ala Gly Pro Phe Asp Tyr Trp Ala
            100                 105                 110

Gln Gly Thr Leu Val Thr Val Ser Ser Ala
        115                 120

<210>  39
<211>  321
<212>  DNA
<213>  Homo Sapiens

<400>  39
gaaacagtga tgacgcagtc tccagccacc ctgtctgtgt ctccagggga aagagtcacc      60

ctctcctgca gggccagtca gagtgttatc agcagcttag cctggtacca gcagaaacct     120

ggccaggctc ccaggctcct catctatggt gcatccacca gggccactgg tatcccagcc     180

aggttcagtg gcagtgggtc tgggacagag ttcactctca ccatcagcag cctgcagtct     240

gaagattttg cagtttatta ctgtcagcag tataataatt ggtggacgtt cggccaaggg     300

accaaggtgg aaatcaaacg a                                               321

<210>  40
<211>  107
<212>  PRT
<213>  Homo sapiens

<400>  40

Glu Thr Val Met Thr Gln Ser Pro Ala Thr Leu Ser Val Ser Pro Gly
1               5                   10                  15

Glu Arg Val Thr Leu Ser Cys Arg Ala Ser Gln Ser Val Ile Ser Ser
            20                  25                  30

Leu Ala Trp Tyr Gln Gln Lys Pro Gly Gln Ala Pro Arg Leu Leu Ile
        35                  40                  45

Tyr Gly Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Asn Trp Trp Thr
                85                  90                  95

Phe Gly Gln Gly Thr Lys Val Glu Ile Lys Arg
            100                 105

## Description

This invention relates to compositions which possess anti-angiogenic activity and are accordingly useful in methods of treatment of disease states associated with angiogenesis in the animal or human body. More specifically the invention concerns a combination of an antagonist of the biological activity of Angiopoietin-2 and an antagonist of the biological activity of VEGF-A, and/or KDR, and/or Flt1, and uses of such antagonists. Such combinations are also useful for the treatment of diseases associated with the activity of Angiopoietin-2 and VEGF-A, and/or KDR, and/or Flt1.

Angiogenesis, the formation of new blood vessels from existing vasculature, is a complex biological process required for the formation and physiological functions of virtually all the organs. It is an essential element of embryogenesis, normal physiological growth, repair and pathological processes such as tumour expansion. Normally, angiogenesis is tightly regulated by the local balance of angiogenic and angiostatic factors in a multi-step process involving vessel sprouting, branching and tubule formation by endothelial cells (involving processes such as activation of endothelial cells (ECs), vessel destabilisation, synthesis and release of degradative enzymes, EC migration, EC proliferation, EC organisation and differentiation and vessel maturation).

In the adult, physiological angiogenesis is largely confined to wound healing and several components of female reproductive function and embryonic development. In disease-related angiogenesis which includes any abnormal, undesirable or pathological angiogenesis, the local balance between angiogenic and angiostatic factors is dysregulated leading to inappropriate and/or structurally abnormal blood vessel formation. Pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacology. Science. 16: 57-66; Folkman, 1995, Nature Medicine 1: 27-31). In cancer, growth of primary and secondary tumours beyond 1-2 mm³ requires angiogenesis (Folkman, J. New England Journal of Medicine 1995; 33, 1757-1763).

Many signal transduction systems have been implicated in the regulation of angiogenesis and a number of factors are known modulators of EC response *in vitro* and blood vessel growth *in vivo.* The receptor tyrosine kinases (RTKs) are important transmitters of biochemical signals across the plasma membrane of cells. These transmembrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity which leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified.

VEGF is believed to be an important stimulator of both normal and disease-related angiogenesis (Jakeman, et al. 1993 Endocrinology: 133,848-859; Kolch, et al. 1995 Breast Cancer Research and Treatment: 36,139-155) and vascular permeability (Connolly, et al. 1989 J. Biol. Chem: 264,20017-20024). Antagonism of VEGF action by sequestration of VEGF with antibody can result in inhibition of tumour growth (Kim, et al. 1993 Nature: 362,841-844). Heterozygous disruption of the VEGF gene resulted in fatal deficiencies in vascularisation (Carmeliet, et al. 1996 Nature 380:435-439; Ferrara, et al. 1996 Nature 380:439-442).

VEGF is the most potent and ubiquitous vascular growth factor known. Prior to identification of the role of VEGF as a secreted mitogen for endothelial cells, it was identified as a vascular permeability factor, highlighting VEGF's ability to control many distinct aspects of endothelial cell behaviour, including proliferation, migration, specialization and survival (Ruhrberg, 2003 BioEssays 25:1052-1060). VEGF, also known as VEGF-A, was the first member of the VEGF family of structurally related dimeric glycoproteins belonging to the platelet-derived growth factor superfamily to be identified. Beside the founding member, the VEGF family includes VEGF-B, VEGF-C, VEGF-D, VEGF-E, placental growth factor (PIGF) and endocrine gland-derived VEGF (EG-VEGF). Active forms of VEGF are synthesised either as homodimers or heterodimers with other VEGF family members. VEGF-A exists in six isoforms generated by alternative splicing; VEGF₁₂₁, VEGF₁₄₅, VEGF₁₆₅, VEGF₁₈₃, VEGF₁₈₉ and VEGF₂₀₆. These isoforms differ primarily in their bioavailability, with VEGF₁₆₅ being the predominant isoform (Podar, et al. 2005 Blood 105(4):1383-1395). The regulation of splicing during embryogenesis to produce stage- and tissue-specific ratios of the various isoforms creates rich potential for distinct and context dependent behaviour of endothelial cells in response to VEGF.

Members of the VEGF family are known to bind with different affinities to three related receptor tyrosine kinases; VEGFR1 (the fins-like tyrosine kinase receptor, Flt or Flt1), VEGFR2 (the kinase insert domain-containing receptor, KDR (also referred to as Flk-1)), and VEGFR3 (another fms-like tyrosine kinase receptor, Flt4). Two of these related RTKs, Fltl and KDR, have been shown to bind VEGF with high affinity (De Vries et al, 1992, Science 255: 989-991; Terman et al, 1992, Biochem. Biophys. Res. Comm. 1992, 187: 1579-1586). Binding of VEGF to these receptors expressed in heterologous cells has been associated with changes in the tyrosine phosphorylation status of cellular proteins and calcium fluxes.

Knock-out mouse studies have shown that disruptions in either Flt1 or KDR, causes death mid-gestation owing to acute vascular defects. However, the phenotypes are distinct; deficiency of KDR leads to a lack of both ECs and a developing haematopoietic system (Shalaby, et al. 1995 Nature 376:62-66), deficiency of Flt1 does not affect hematopoietic progenitors and ECs, but these fail to assemble into functional vessels (Fong, et al. 1995 Nature 376:66-70). Flt4 is expressed extensively in the embryo before being restricted to lymphatic vessels in adults. Flt4 knock-out mice showed an essential role for Flt4 in early development of the cardiovascular system, in remodelling and maturation of the primary vascular networks into larger blood vessels (Dumont, et al. 1998 Science 282:946-949).

In addition to the VEGF family, the angiopoietins are thought to be involved in vascular development and postnatal angiogenesis. The angiopoietins include a naturally occurring agonist, angiopoietin-1 (Angiopoietin-1), as well as a naturally occurring antagonist, angiopoietin-2 (Angiopoietin-2). The role of Angiopoietin-1 is thought to be conserved in the adult, where it is expressed widely and constitutively (Hanahan, Science, 277:48-50 (1997); Zagzag, et al., Exp Neurology, 159:391-400 (1999)). In contrast, Angiopoietin-2 expression is primarily limited to sites of vascular remodeling where it is thought to block the constitutive stabilising or maturing function of Angiopoietin-1, allowing vessels to revert to, and remain in, a plastic state which may be more responsive to sprouting signals (Hanahan, 1997; Holash et al., Oncogene 18:5356-62 (1999); Maisonpierre, 1997). Studies of Angiopoietin-2 expression in disease-related angiogenesis have found many tumour types to show vascular Angiopoietin-2 expression (Maisonpierre et al., Science 277:55-60 (1997)). Functional studies suggest Angiopoietin-2 is involved in tumour angiogenesis and associate Angiopoietin-2 overexpression with increased tumour growth in a mouse xenograft model (Ahmad, et al., Cancer Res., 61:1255-1259 (2001)). Other studies have associated Angiopoietin-2 overexpression with tumour hypervascularity (Etoh, et al., Cancer Res. 61:2145-53 (2001); Tanaka et al., Cancer Res. 62:7124-29 (2002)).

Using homology-based cloning approaches, Valenzuela et al. (1999) identified 2 novel angiopoietins: angiopoietin-3 (Angiopoietin-3) in mouse, and angiopoietin-4 (Angiopoietin-4) in human. Although Angiopoietin-3 and Angiopoietin-4 are more structurally diverged from each other than are the mouse and human versions of Angiopoietin-1 and Angiopoietin-2, they appear to represent the mouse and human counterparts of the same gene locus. Very little is known about the biology of these members of the Angiopoietin family. For example, Angiopoietin-4 is expressed at high levels only in the lung, however no biological actions or signaling pathways activated by Angiopoietin-4 can be found in the literature (Tsigkos, et al., Expert Opin. Investig. Drugs 12(6): 933-941 (2003); Valenzuela, et al., Proc. Natl. Acad. Sci. 96:1904-1909 (1999)). Angiopoietin-4 expression levels are known to increase in response to hypoxia, and endothelial cell growth factors lead to increasing levels of Angiopoietin-4 expression in a glioblastoma cell line and endothelial cells. However, the mechanism of expression regulation, and the resulting effect on physiological and disease-related angiogenesis are unknown (Lee, et al., FASEB J. 18: 1200-1208 (2004).

The angiopoietins were first discovered as ligands for the Tie receptor tyrosine kinase family that is selectively expressed within the vascular endothelium (Yancopoulos et al., Nature 407:242-48 (2000). Angiopoietin-1, Angiopoietin-2, Angiopoietin-3 and Angiopoietin-4 bind primarily to the Tie-2 receptor and so are also known as Tie-2 ligands. Binding of Angiopoietin-1 to Tie-2 induces tyrosine phosphorylation of the receptor via autophosphorylation and subsequently activation of its signalling pathways via signal transduction (Maisonpierre, P. et al. 1997 Science: 277, 55-60). Angiopoietin-2 is a naturally occurring antagonist for Angiopoietin-1 acting through competitive inhibition of Angiopoietin-1-induced kinase activation of the Tie-2 receptor (Hanahan, 1997; Davis et al., Cell 87:1161-69 (1996); Maisonpierre et al., Science 277:55-60 (1997)).

Knock-out mouse studies of Tie-2 and Angiopoietin-1 show similar phenotypes and suggest that Angiopoietin-1 stimulated Tie-2 phosphorylation mediates remodeling and stabilization of developing vessel, promoting blood vessel maturation during angiogenesis and maintenance of endothelial cell-support cell adhesion (Dumont et al., Genes & Development, 8:1897-1909 (1994); Sato, Nature, 376:70-74 (1995); (Thurston, G. et al., 2000 Nature Medicine: 6, 460-463)).

In recent years Angiopoietin-1, Angiopoietin-2 and/or Tie-2 have been proposed as possible anti-cancer therapeutic targets. For example US6166185, US5650490 and US5814464 each disclose anti-Tie-2 ligand and receptor antibodies. Studies using soluble Tie-2 were reported to decrease the number and size of tumours in rodents (Lin, 1997; Lin 1998). Siemester et al. (1999) generated human melanoma cell lines expressing the extracellular domain of Tie-2, injected these into nude mice and reported soluble Tie-2 to result in significant inhibition of tumour growth and tumour angiogenesis. Given both Angiopoietin-1 and Angiopoietin-2 bind to Tie-2, it is unclear from these studies whether Angiopoietin-1, Angiopoietin-2 or Tie-2 would be an attractive target for anti-cancer therapy. However, effective anti-Angiopoietin-2 therapy is thought to be of benefit in treating diseases such as cancer, in which progression is dependant on aberrant angiogenesis where blocking the process can lead to prevention of disease advancement (Folkman, J., Nature Medicine. 1: 27-31 (1995). In addition some groups have reported the use of antibodies that bind to Angiopoietin-2, See, for example, U.S. Patent No. 6,166,185 and U.S. Patent Application Publication No. 2003/0124129 A1. Study of the effect of focal expression of Angiopoietin-2 has shown that antagonising the Angiopoietin-1/Tie-2 signal loosens the tight vascular structure thereby exposing ECs to activating signals from angiogenesis inducers, e.g. VEGF (Hanahan, 1997). This pro-angiogenic effect resulting from inhibition of Angiopoietin-1 indicates that anti-Angiopoietin-1 therapy would not be an effective anti-cancer treatment.

International publication number WO200197850 describes the combination of functional interference with VEGF/VEGF receptor systems and Angiopoietin/Tie receptor systems for inhibition of vascularisation and of tumour growth. The broad scope includes any conceivable combination of functional interference of any component of the VEGF/VEGF receptor systems and Angiopoietin/Tie receptor system; that is any one of Fltl, KDR, Flt4, VEGF-A, VEGF-B, VEGF-C, VEGF-D, VEGF-E, PIGF or EG-VEGF combined with functional interference of any one of Angiopoietin-1, Angiopoietin-2, Angiopoietin-3, Angiopoietin-4 or Tie-2.

The application suggests that functional interference may be achieved by
i) compounds which inhibit receptor tyrosine kinase activity,
ii) compounds which inhibit ligand binding to receptors,
iii) compounds which inhibit activation of intracellular pathways of the receptor,
iv) compounds which inhibit or activate expression of a ligand or of a receptor of the VEGF or Tie receptor system,
v) delivery systems such as antibodies, ligands, high-affinity binding oligonucleotides or oligopeptides, or liposomes which target cytotoxic agents or coagulation-inducing agents to the endothelium via recognition of VEGF/VEGF receptor or Angiopoietin/Tie receptor systems, or
vi) delivery systems such as antibodies, ligands, high-affinity binding oligonucleotides or oligopeptides, or liposomes , which are targeted to the endothelium and induce necrosis or apoptosis.

Further broadening the claimed scope the application further states that the compound comprised by combinations of the present invention can be a small molecular weight substance, an oligonucleotide, an oligopeptide, a recombinant protein, an antibody, or conjugates or fusion proteins thereof. The inclusion of vast numbers of optional combinations does not teach the utility/selection of particular combinations.

Although WO200197850 claims a very large scope, exemplification of the invention is limited to combinations of the extracellular ligand-neutralising domain of human Tie-2 receptor tyrosine kinase (sTie-2) and A or B. The latter may be:
A. VEGF receptor tyrosine kinase inhibitor (4-Chlorophenyl)[4-(4-pyridylmethyl)-phthalazin-1-yl] ammonium hydrogen succinate (Wood et al., Cancer Res. 60 2178-2189, 2000), or
B. Anti-VEGF antibody; either VEGF-A-neutralising monoclonal antibody 4301-42-35 (Schlaeppi et al., J. Cancer Res. Clin. Oncol. 125, 336-342, 1999), or single chain antibody (scFv) specifically recognizing the human VEGF-A/VEGF receptor I complex (WO9919361).

There is no exemplification of the remainder of the broad scope of the application. In particular, there is no exemplification other than the use of sTie-2 to achieve functional interference with the Angiopoietin/Tie receptor system. It is therefore unclear to the skilled person what other combinations, from the very large range of possible permutations, would be therapeutically effective.

The present invention relates to a combination of an antagonist of the biological activity of Angiopoietin-2 and an antagonist of the biological activity of VEGF-A, and/or KDR, and/or Flt1, and uses of such combinations.

According to one aspect of the invention there is provided a combination of an antagonist of the biological activity of Angiopoietin-2 and an antagonist of the biological activity of
i. VEGF-A, and/or
ii. KDR, and/or
iii. Flt1.

In one embodiment, there is provided a combination as described above, wherein the antagonist of the biological activity of Angiopoietin-2 is an antibody. Preferably the antagonist of Angiopoietin-2 is a monoclonal antibody. More preferably the antagonist of Angiopoietin-2 is a fully human monoclonal antibody. More preferably the fully human monoclonal antibody binds to the same epitope as any one of fully human monoclonal antibody; 3.31.2, 5.16.3, 5.86.1, 5.88.3, 3.3.2, 5.103.1, 5.101.1, 3.19.3, 5.28.1, 5.78.3. Most preferably the fully human monoclonal antibody is selected from any one of; 3.31.2, or 5.16.3, or 5.86.1, or 5.88.3, or 3.3.2, or 5.103.1, or 5.101.1, or 3.19.3, or 5.28.1, or 5.78.3.

In another embodiment, there is provided a combination as described above, wherein the antagonist of the biological activity of Angiopoietin-2 may not bind to the ATP-binding site of Tie-2.

In another embodiment, there is provided a combination as described above, wherein the antagonist of the biological activity of Angiopoietin-2 is not sTie-2.

In another embodiment there is provided a combination as described above, wherein the antagonist of the biological activity of KDR is an antibody. Preferably the antagonist is a monoclonal antibody. More preferably the antagonist is a fully human monoclonal antibody.

In another embodiment there is provided a combination as described above, wherein the antagonist of the biological activity of Flt1 is an antibody. Preferably the antagonist is a monoclonal antibody. More preferably the antagonist is a fully human monoclonal antibody.

In another embodiment there is provided a combination as described above, wherein the antagonist of the biological activity of VEGF-A is an antibody. Preferably the antagonist is a monoclonal antibody. The monoclonal antibody may be DC101 (Imclone). More preferably the antagonist is a fully human monoclonal antibody. Most preferably the antagonist of the biological activity of VEGF-A is Avastin (bevacizumab) (Rosen LS., Cancer Control 9 (suppl 2):36-44, 2002), CDP791 (Celltech) or IMC1121b (Imclone).

In another embodiment there is provided a combination as described above, wherein the antagonist of the biological activity of KDR is a compound. In an alternative embodiment there is provided a combination as described above, wherein the antagonist of the biological activity of Flt1 is a compound. Preferably the antagonist is a tyrosine kinase inhibitor. More preferably the tyrosine kinase inhibitor is selected from Zactima™ (ZD6474 (Wedge SR et al. ZD6474 inhibits VEGF signalling, angiogenesis and tumour growth following oral administration. Cancer Research 2002; 62:4645-4655)), AZD2171 (Wedge SR et al. AZD2171: A highly potent, orally bioavailable, vascular endothelial growth factor receptor-2 tyrosine kinase inhibitor for the treatment of cancer. Cancer Research 2005; 65:4389-4400), SU11248 (Sutent, Pfizer), SU14813 (Pfizer), Vatalanib (Novartis), BAY43-9006 (sorafenib, Bayer), XL-647 (Exelixis), XL-999 (Exelixis), AG-013736 (Pfizer), AMG706 (Amgen), BIBF1120 (Boehringer), TSU68 (Taiho), GW786034, AEE788 (Novartis), CP-547632 (Pfizer), KRN 951 (Kirin), CHIR258 (Chiron), CEP-7055 (Cephalon), OSI-930 (OSI Pharmaceuticals), ABT-869 (Abbott), E7080 (Eisai), ZK-304709 (Schering), BAY57-9352 (Bayer), L-21649 (Merck), BMS582664 (BMS), XL-880 (Exelixis), XL-184 (Exelixis) or XL-820 (Exelixis). More preferably the tyrosine kinase inhibitor is selected from Zactima™ or AZD2171.

For the avoidance of doubt, an antagonist of the biological activity of KDR may inhibitor other tyrosine kinases in addition to KDR, for example Flt1, EGFR or PDGFR. In one embodiment an antagonist of the biological activity of KDR is a KDR signalling inhibitor. In another embodiment an antagonist of the biological activity of KDR is an inhibitor of KDR signalling, but not an inhibitor of EGFR.

According to another aspect of the invention there is provided a pharmaceutical composition comprising a combination as described hereinabove.

According to another aspect of the invention there is provided the use of a combination as described hereinabove for the manufacture of a medicament for the treatment of disease-related angiogenesis.

A combination of an antagonist of the biological activity of Angiopoietin-2 and an antagonist of the biological activity of VEGF-A, and/or KDR, and/or Flt1 can be administered alone, or can be administered in combination with additional antibodies or chemotherapeutic drugs or radiation therapy.

According to another aspect of the invention there is provided a method of antagonising the biological activity of Angiopoietin-2 and the biological activity of any one of; VEGF-A, and/or KDR, and/or Fltl, comprising administering a combination as described hereinabove. Preferably the method comprises selecting an animal in need of treatment for disease-related angiogenesis, and administering to said animal a therapeutically effective dose of a combination of an antagonist of the biological activity of Angiopoietin-2 and an antagonist of the biological activity of VEGF-A, and/or KDR, and/or Flt1.

According to another aspect of the invention there is provided a method of treating disease-related angiogenesis in a mammal comprising administering a therapeutically effective amount of a combination as described hereinabove. Preferably the method comprises selecting an animal in need of treatment for disease-related angiogenesis, and administering to said animal a therapeutically effective dose of a combination of an antagonist of the biological activity of Angiopoietin-2 and an antagonist of the biological activity of VEGF-A, and/or KDR, and/or Flt1.

According to another aspect of the invention there is provided a method of treating cancer in a mammal comprising a therapeutically effective amount of a combination as described hereinabove. Preferably the method comprises selecting a mammal in need of treatment for disease-related angiogenesis, and administering to said mammal a therapeutically effective dose of a combination of an antagonist of the biological activity of Angiopoietin-2 and an antagonist of the biological activity of VEGF-A, and/or KDR, and/or Flt1.

In a preferred embodiment the present invention is particularly suitable for use in antagonizing the biological activity of Angiopoietin-2 and the biological activity of VEGF-A, and/or KDR, and/or Flt1, in patients with a tumour which is dependent alone, or in part, on Angiopoietin-2 and VEGF-A, and/or KDR, and/or Flt1.

According to another aspect of the invention there is provided a combination of the invention additionally comprising antiproliferative/antineoplastic drugs and combinations thereof, as used in medical oncology, such as alkylating agents (for example cis-platin, carboplatin, oxaliplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, gemcitabine, capecitabine, methotrexate, pemetrexed, cytosine arabinoside and hydroxyurea, or, for example, one of the preferred antimetabolites disclosed in European Patent Application No. 562734 such as (2S)-2- {o-fluoro-p-[N-{2,7-dimethyl-4-oxo-3,4-dihydroquinazolin-6-ylmethyl)-N-(prop-2-ynyl)amino]benzamido}-4-(tetrazol-5-yl)butyric acid); combinations which comprise an alkylating agent and an antimetabolite (for example Folfox, which comprises fluorouracil, leucovorin and oxaliplatin); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, irinotecan, amsacrine, topotecan and camptothecin). In a preferred embodiment there is provided a combination of the invention additionally comprising Folfox.

In a preferred embodiment a combination of the invention further comprises a protective agent, for example an agent which acts to prevent anaemia or to reduce the side effects from antiproliferative/antineoplastic drugs. Preferably the protective agent is a reduced form of folic acid, preferably leucovorin.

Combinations of the invention are expected to inhibit disease-related angiogenesis and thereby act as a potent therapy for various angiogenesis-related diseases.

In embodiments of the invention comprising an antibody, a combination may be administered to a patient, followed by administration of a clearing agent. Preferably the clearing agent can remove excess circulating antibody from the blood.

The invention further comprises processes for the preparation of combinations of the invention.

According to a further aspect of the present invention there is provided a kit comprising a combination of an antagonist of the biological activity of Angiopoietin-2 and an antagonist of the biological activity of VEGF-A, and/or KDR, and/or Flt1.

According to a further aspect of the present invention there is provided a kit comprising:
a) an antagonist of the biological activity of Angiopoietin-2 in a first unit dosage form;
b) an antagonist of the biological activity of VEGF-A, and/or KDR, and/or Flt1 in a second unit dosage form; and
c) a container means for containing said first and second dosage forms.

According to a further aspect of the present invention there is provided a kit comprising:
a) an antagonist of the biological activity of Angiopoietin-2, together with a pharmaceutically acceptable excipient or carrier, in a first unit dosage form;
b) an antagonist of the biological activity of VEGF-A, and/or KDR, and/or Flt1 together with a pharmaceutically acceptable excipient or carrier, in a second unit dosage form; and
c) a container means for containing said first and second dosage forms.

In another embodiment, the invention provides an article of manufacture including a container. The container includes a combination of an antagonist of the biological activity of Angiopoietin-2 and an antagonist of the biological activity of VEGF-A, and/or KDR, and/or Flt1, and a package insert or label indicating that the combination can be used to treat angiogenesis-related diseases associated with the activity and/or overexpression of Angiopoietin-2 and VEGF-A, and/or KDR, and/or Flt1.

According to a further aspect of the present invention there is provided a therapeutic combination treatment comprising the administration of an effective amount of an antagonist of the biological activity of Angiopoietin-2 or a pharmaceutically acceptable salt thereof, optionally together with a pharmaceutically acceptable excipient or carrier, and the simultaneous, sequential or separate administration of an effective amount of an antagonist of the biological activity of VEGF-A, and/or KDR, and/or Flt1 or a pharmaceutically acceptable salt thereof, wherein the latter may optionally be administered together with a pharmaceutically acceptable excipient or carrier, to a warm-blooded animal such as a human in need of such therapeutic treatment.

A combination treatment of the present invention as defined herein may be achieved by way of the simultaneous, sequential or separate administration of the individual components of said treatment. A combination treatment as defined herein may be applied as a sole therapy or may involve additional surgery or radiotherapy or an additional chemotherapeutic agent in addition to a combination treatment of the invention.

The dosage of a combination formulation for a given patient will be determined by the attending physician taking into consideration various factors known to modify the action of drugs including severity and type of disease, body weight, sex, diet, time and route of administration, other medications and other relevant clinical factors. Therapeutically effective dosages may be determined by either *in vitro* or *in vivo* methods.

An effective amount of a combination, described herein, to be employed therapeutically will depend, for example, upon the therapeutic objectives, the route of administration, and the condition of the patient. Accordingly, it is preferred for the therapist to titer the dosage and modify the route of administration as required to obtain the optimal therapeutic effect. A typical daily dosage might range from about 0.001mg/kg to up to 100mg/kg or more, depending on the factors mentioned above. Typically, the clinician will administer the therapeutic antibody until a dosage is reached that achieves the desired effect. The progress of this therapy is easily monitored by conventional assays or as described herein.

The route of antibody administration is in accord with known methods, e.g., injection or infusion by intravenous, intraperitoneal, intracerebral, intramuscular, intraocular, intraarterial, intrathecal, inhalation or intralesional routes, or by sustained release systems as noted below. The antibody is preferably administered continuously by infusion or by bolus injection.

An effective amount of antibody to be employed therapeutically will depend, for example, upon the therapeutic objectives, the route of administration, and the condition of the patient. Accordingly, it is preferred that the therapist titer the dosage and modify the route of administration as required to obtain the optimal therapeutic effect. Typically, the clinician will administer antibody until a dosage is reached that achieves the desired effect. The progress of this therapy is easily monitored by conventional assays or by the assays described herein.

A combination as described herein may be in a form suitable for oral administration, for example as a tablet or capsule, for nasal administration or administration by inhalation, for example as a powder or solution, for parenteral injection (including intravenous, subcutaneous, intramuscular, intravascular or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention a combination treatment may be delivered endoscopically, intratracheally, intralesionally, percutaneously, intravenously, subcutaneously, intraperitoneally or intratumourally. Preferably a combination of the invention is administered orally. In general the combinations described herein may be prepared in a conventional manner using conventional excipients. A combination of the present invention is advantageously presented in unit dosage form.

Antibodies, as described herein, can be prepared in a mixture with a pharmaceutically acceptable carrier. This therapeutic composition can be administered intravenously or through the nose or lung, preferably as a liquid or powder aerosol (lyophilized). The composition may also be administered parenterally or subcutaneously as desired. When administered systemically, the therapeutic composition should be sterile, pyrogen-free and in a parenterally acceptable solution having due regard for pH, isotonicity, and stability. These conditions are known to those skilled in the art. Briefly, dosage formulations of the compounds described herein are prepared for storage or administration by mixing the compound having the desired degree of purity with physiologically acceptable carriers, excipients, or stabilizers. Such materials are non-toxic to the recipients at the dosages and concentrations employed, and include buffers such as TRIS HCl, phosphate, citrate, acetate and other organic acid salts; antioxidants such as ascorbic acid; low molecular weight (less than about ten residues) peptides such as polyarginine, proteins, such as serum albumin, gelatin, or immunoglobulins; hydrophilic polymers such as polyvinylpyrrolidinone; amino acids such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium and/or nonionic surfactants such as TWEEN, PLURONICS or polyethyleneglycol.

Embodiments of the invention include sterile pharmaceutical formulations of antibodies that are useful as treatments for diseases. Such formulations would inhibit the biological activity of the antigen, thereby effectively treating disease conditions where, for example, serum or tissue antigen is abnormally elevated. The antibodies preferably possess adequate affinity to potently neutralize the antigen, and preferably have an adequate duration of action to allow for infrequent dosing in humans. A prolonged duration of action will allow for less frequent and more convenient dosing schedules by alternate parenteral routes such as subcutaneous or intramuscular injection.

Sterile formulations can be created, for example, by filtration through sterile filtration membranes, prior to or following lyophilization and reconstitution of the antibody. The antibody ordinarily will be stored in lyophilized form or in solution. Therapeutic antibody compositions generally are placed into a container having a sterile access port, for example, an intravenous solution bag or vial having an adapter that allows retrieval of the formulation, such as a stopper pierceable by a hypodermic injection needle.

Sterile compositions for injection can be formulated according to conventional pharmaceutical practice as described in Remington: The Science and Practice of Pharmacy (20th ed, Lippincott Williams & Wilkens Publishers (2003)). For example, dissolution or suspension of the active compound in a vehicle such as water or naturally occurring vegetable oil like sesame, peanut, or cottonseed oil or a synthetic fatty vehicle like ethyl oleate or the like may be desired. Buffers, preservatives, antioxidants and the like can be incorporated according to accepted pharmaceutical practice.

Combinations of the invention could be delivered as sustained release formulations. Suitable examples of sustained-release preparations include semipermeable matrices of solid hydrophobic polymers containing the polypeptide, which matrices are in the form of shaped articles, films or microcapsules. Examples of sustained-release matrices include polyesters, hydrogels (e.g., poly(2-hydroxyethyl-methacrylate) as described by Langer et al., J. Biomed Mater. Res., (1981) 15:167-277 and Langer, Chem. Tech., (1982) 12:98-105, or poly(vinylalcohol)), polylactides (U.S. Pat. No. 3,773,919, EP 58,481), copolymers of L-glutamic acid and gamma ethyl-L-glutamate (Sidman et al., Biopolymers, (1983) 22:547-556), non-degradable ethylene-vinyl acetate (Langer *et al., supra*), degradable lactic acid-glycolic acid copolymers such as the LUPRON Depot™ (injectable microspheres composed of lactic acid-glycolic acid copolymer and leuprolide acetate), and poly-D-(-)-3-hydroxybutyric acid (EP 133,988).

While polymers such as ethylene-vinyl acetate and lactic acid-glycolic acid enable release of molecules for over 100 days, certain hydrogels release proteins for shorter time periods. When encapsulated proteins remain in the body for a long time, they may denature or aggregate as a result of exposure to moisture at 37°C, resulting in a loss of biological activity and possible changes in immunogenicity. Rational strategies can be devised for protein stabilization depending on the mechanism involved. For example, if the aggregation mechanism is discovered to be intermolecular S-S bond formation through disulfide interchange, stabilization may be achieved by modifying sulfhydryl residues, lyophilizing from acidic solutions, controlling moisture content, using appropriate additives, and developing specific polymer matrix compositions.

Sustained-released compositions also include preparations of crystals of the antibody suspended in suitable formulations capable of maintaining crystals in suspension. These preparations when injected subcutaneously or intraperitonealy can produce a sustained release effect. Other compositions also include liposomally entrapped antibodies. Liposomes containing such antibodies are prepared by methods known per se: U.S. Pat No. DE 3,218,121; Epstein et al., Proc. Natl. Acad. Sci. USA, (1985) 82:3688-3692; Hwang et al., Proc. Natl. Acad. Sci. USA, (1980) 77:4030-4034; EP 52,322; EP 36,676; EP 88,046; EP 143,949; 142,641; Japanese patent application 83-118008; U.S. Pat. Nos. 4,485,045 and 4,544,545; and EP 102,324.

It will be appreciated that administration of therapeutic entities in accordance with the compositions and methods herein will be administered with suitable carriers, excipients, and other agents that are incorporated into formulations to provide improved transfer, delivery, tolerance, and the like. These formulations include, for example, powders, pastes, ointments, jellies, waxes, oils, lipids, lipid (cationic or anionic) containing vesicles (such as Lipofectin™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. Any of the foregoing mixtures may be appropriate in treatments and therapies in accordance with the present invention, provided that the active ingredient in the formulation is not inactivated by the formulation and the formulation is physiologically compatible and tolerable with the route of administration. *See also* Baldrick P. "Pharmaceutical excipient development: the need for preclinical guidance." Regul. Toxicol. Pharmacol. 32(2):210-8 (2000), Wang W. "Lyophilization and development of solid protein pharmaceuticals." Int. J. Pharm. 203(1-2):1-60 (2000), Charman WN "Lipids, lipophilic drugs, and oral drug delivery-some emerging concepts." J Pharm Sci .89(8):967-78 (2000), Powell et al. "Compendium of excipients for parenteral formulations" PDA J Pharm Sci Technol. 52:238-311 (1998) and the citations therein for additional information related to formulations, excipients and carriers well known to pharmaceutical chemists.

The manufacture of monoclonal antibodies of predefined specificity by means of permanent tissue culture cell lines was first described in 1975 (Kohler, G., & Milstein, C., Nature 256, 495-497, 1975). Fusion of a mouse myeloma and mouse spleen cells from an immunised donor created a cell line which secreted anti-sheep red blood cell (SRBC) antibodies. Subsequent developments mean it is now possible to derive human antibodies by in vitro methods. Suitable examples include but are not limited to phage display (CAT, Morphosys, Dyax, Biosite/Medarex, Xoma, Symphogen, Alexion (formerly Proliferon), Affimed) ribosome display (CAT), yeast display, and the like.

Antibodies, as described herein, were prepared through the utilization of the XenoMouse^{®} technology, as described below. Such mice, then, are capable of producing human immunoglobulin molecules and antibodies and are deficient in the production of murine immunoglobulin molecules and antibodies. Technologies utilized for achieving the same are disclosed in the patents, applications, and references disclosed herein. In particular, however, a preferred embodiment of transgenic production of mice and antibodies therefrom is disclosed in U.S. Patent Application Serial No. 08/759,620, filed December 3, 1996 and International Patent Application Nos. WO 98/24893, published June 11, 1998 and WO 00/76310, published December 21, 2000, the disclosures of which are hereby incorporated by reference. See also Mendez et al. Nature Genetics 15:146-156 (1997), the disclosure of which is hereby incorporated by reference.

Through the use of such technology, fully human monoclonal antibodies to a variety of antigens have been produced. Essentially, XenoMouse^{®} lines of mice are immunized with an antigen of interest, lymphatic cells (such as B-cells) are recovered from the hyper-immunized mice, and the recovered lymphocytes are fused with a myeloid-type cell line to prepare immortal hybridoma cell lines. These hybridoma cell lines are screened and selected to identify hybridoma cell lines that produced antibodies specific to the antigen of interest. Provided herein are methods for the production of multiple hybridoma cell lines that produce antibodies. Further, provided herein are characterization of the antibodies produced by such cell lines, including nucleotide and amino acid sequence analyses of the heavy and light chains of such antibodies.

Alternatively, instead of being fused to myeloma cells to generate hybridomas, B cells can be directly assayed. For example, CD19+ B cells can be isolated from hyperimmune XenoMouse® mice and allowed to proliferate and differentiate into antibody-secreting plasma cells. Antibodies from the cell supernatants are then screened by ELISA for reactivity against the immunogen. The supernatants might also be screened for immunoreactivity against fragments of the immunogen to further map the different antibodies for binding to domains of functional interest on the immunogen. The antibodies may also be screened against other related human proteins and against the rat, mouse, and non-human primate, such as cynomolgus monkey, orthologues of the immunogen, to determine species cross-reactivity. B cells from wells containing antibodies of interest may be immortalized by various methods including fusion to make hybridomas either from individual or from pooled wells, or by infection with Epstein Barr Virus or transfection by known immortalizing genes and then plating in suitable medium. Alternatively, single plasma cells secreting antibodies with the desired specificities are then isolated using antigen-specific hemolytic plaque assays (see for example Babcook et al., Proc. Natl. Acad. Sci. USA 93:7843-48 (1996)). Cells targeted for lysis are preferably sheep red blood cells (SRBCs) coated with the antigen.

In the presence of a B-cell culture containing plasma cells secreting the immunoglobulin of interest and complement, the formation of a plaque indicates specific antigen-mediated lysis of the sheep red blood cells surrounding the plasma cell of interest. The single antigen-specific plasma cell in the center of the plaque can be isolated and the genetic information that encodes the specificity of the antibody is isolated from the single plasma cell. Using reverse-transcription followed by polymerase chain reaction (RT-PCR), the DNA encoding the heavy and light chain variable regions of the antibody can be cloned. Such cloned DNA can then be further inserted into a suitable expression vector, preferably a vector cassette such as a pcDNA, more preferably such a pcDNA vector containing the constant domains of immunglobulin heavy and light chain. The generated vector can then be transfected into host cells, e.g., HEK293 cells, CHO cells, and cultured in conventional nutrient media modified as appropriate for inducing transcription, selecting transformants, or amplifying the genes encoding the desired sequences.

In general, antibodies produced by the fused hybridomas were human IgG2 heavy chains with fully human kappa or lambda light chains. Antibodies described herein possess human IgG4 heavy chains as well as IgG2 heavy chains. Antibodies can also be of other human isotypes, including IgG1. The antibodies possessed high affinities, typically possessing a Kd of from about 10⁻⁶ through about 10⁻¹² M or below, when measured by solid phase and solution phase techniques.

The generation of human antibodies from mice in which, through microcell fusion, large pieces of chromosomes, or entire chromosomes, have been introduced, is described in European Patent Application Nos. 773 288 and 843 961, the disclosures of which are hereby incorporated by reference. Additionally, KM™ mice, which are the result of cross-breeding of Kirin's Tc mice with Medarex's minilocus (Humab) mice have been generated. These mice possess the human IgH transchromosome of the Kirin mice and the kappa chain transgene of the Genpharm mice (Ishida et al., Cloning Stem Cells, (2002) 4:91-102).

As will be appreciated, antibodies can be expressed in cell lines other than hybridoma cell lines. Sequences encoding particular antibodies can be used to transform a suitable mammalian host cell. Transformation can be by any known method for introducing polynucleotides into a host cell, including, for example packaging the polynucleotide in a virus (or into a viral vector) and transducing a host cell with the virus (or vector) or by transfection procedures known in the art, as exemplified by U.S. Patent Nos. 4,399,216, 4,912,040, 4,740,461, and 4,959,455 (which patents are hereby incorporated herein by reference). The transformation procedure used depends upon the host to be transformed. Methods for introducing heterologous polynucleotides into mammalian cells are well known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are well known in the art and include many immortalized cell lines available from the American Type Culture Collection (ATCC), including but not limited to Chinese hamster ovary (CHO) cells, HeLa cells, baby hamster kidney (BHK) cells, monkey kidney cells (COS), human hepatocellular carcinoma cells (e.g., Hep G2), human epithelial kidney 293 cells, and a number of other cell lines. Cell lines of particular preference are selected through determining which cell lines have high expression levels and produce antibodies with constitutive antigen binding properties.

Unless otherwise defined, scientific and technical terms used herein shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art.

Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. *See e.g.,* Sambrook et al. Molecular Cloning: A Laboratory Manual (3rd ed., Cold Spring Harbor Laboratory Press, Cold Spring Harbor, N.Y. (2001)), which is incorporated herein by reference. The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

The following terms, unless otherwise indicated, shall be understood to have the following meanings:
An antagonist may be a polypeptide, nucleic acid, carbohydrate, lipid, small molecular weight compound, an oligonucleotide, an oligopeptide, RNA interference (RNAi), antisense, a recombinant protein, an antibody, or conjugates or fusion proteins thereof. For a review of RNAi see Milhavet O, Gary DS, Mattson MP. (Pharmacol Rev. 2003 Dec;55(4):629-48. Review.) and antisense see Opalinska JB, Gewirtz AM. (Sci STKE. 2003 Oct 28;2003(206):pe47.)

An antagonist of Angiopoietin-2 may be any antagonist of the biological activity of Angipoietin-2, including antagonists that antagonise the biological activity of Angiopoietin-2 and other angiopoietins including Angiopoietin-1, Angiopoietin-3 and/or Angiopoietin-4. An Angiopoietin-2 antagonist may bind to the ligand alone, or to the ligand when the ligand is bound to its receptor.

An antagonist of VEGF-A may be any antagonist of the biological activity of VEGF-A, wherein the antagonist may bind to the ligand alone, or to the ligand when the ligand is bound to its receptor. The antagonist may prevent VEGF-A mediated Flt1 or KDR signal transduction, thereby inhibiting angiogenesis. The mechanism of action of this inhibition may include binding of the antagonist to VEGF-A and inhibiting the binding of VEGF-A to its receptor, either Fltl or KDR. Alternatively the antagonist may bind to VEGF-A when VEGF-A is associated with a receptor, either Flt1 or KDR, and thereby prevent VEGF-A mediated Flt1 or KDR signal transduction. Alternatively the antagonist may enhance clearance of VEGF-A therein lowering the effective concentration of VEGF-A for binding to Flt1 or KDR.

A composition is preferably a pharmaceutical composition comprising one or more antagonists. The antagonists of the composition may be administered separately, sequentially or concurrently.

Disease-related angiogenesis may be any abnormal, undesirable or pathological angiogenesis, for example tumor-related angiogenesis. Angiogenesis-related diseases include, but are not limited to, non-solid tumours such as leukaemia, multiple myeloma, haematologic malignancies or lymphoma, and also solid tumours and their metastases such as melanoma, non-small cell lung cancer, glioma, hepatocellular (liver) carcinoma, glioblastoma, carcinoma of the thyroid, bile duct, bone, gastric, brain/CNS, head and neck, hepatic, stomach, prostrate, breast, renal, testicular, ovarian, skin, cervical, lung, muscle, neuronal, oesophageal, bladder, lung, uterine, vulval, endometrial, kidney, colorectal, pancreatic, pleural/peritoneal membranes, salivary gland, and epidermoid tumours.

Excessive vascular growth also contributes to numerous non-neoplastic disorders. These non-neoplastic angiogenesis-related diseases include: atherosclerosis, haemangioma, haemangioendothelioma, angiofibroma, vascular malformations (e.g. Hereditary Hemorrhagic Teleangiectasia (HHT), or Osler-Weber syndrome), warts, pyogenic granulomas, excessive hair growth, Kaposis' sarcoma, scar keloids, allergic oedema, psoriasis, dysfunctional uterine bleeding, follicular cysts, ovarian hyperstimulation, endometriosis, respiratory distress, ascites, peritoneal sclerosis in dialysis patients, adhesion formation result from abdominal surgery, obesity, rheumatoid arthritis, synovitis, osteomyelitis, pannus growth, osteophyte, hemophilic joints, inflammatory and infectious processes (e.g. hepatitis, pneumonia, glomerulonephritis), asthma, nasal polyps, liver regeneration, pulmonary hypertension, retinopathy of prematurity, diabetic retinopathy, age-related macular degeneration., leukomalacia, neovascular glaucoma, corneal graft neovascularization, trachoma, thyroiditis, thyroid enlargement, and lymphoproliferative disorders.

A compound refers to any small molecular weight compound with a molecular weight of less than 2000 Daltons.

The term 'antibody' refers to a polypeptide or group of polypeptides that are comprised of at least one binding domain that is formed from the folding of polypeptide chains having three-dimensional binding spaces with internal surface shapes and charge distributions complementary to the features of an antigenic determinant of an antigen. An antibody typically has a tetrameric form, comprising two identical pairs of polypeptide chains, each pair having one "light" and one "heavy" chain. The variable regions of each light/heavy chain pair form an antibody binding site. An antibody may be oligoclonal, a polyclonal antibody, a monoclonal antibody, a chimeric antibody, a CDR-grafted antibody, a multi-specific antibody, a bi-specific antibody, a catalytic antibody, a chimeric antibody, a humanized antibody, a fully human antibody, an anti-idiotypic antibody and antibodies that can be labeled in soluble or bound form as well as fragments, variants or derivatives thereof, either alone or in combination with other amino acid sequences provided by known techniques. An antibody may be from any species. The term antibody also includes binding fragments of the antibodies of the invention; exemplary fragments include Fv, Fab , Fab', single stranded antibody (svFC), dimeric variable region (Diabody) and disulphide stabilized variable region (dsFv).

The term "neutralizing" when referring to an antibody relates to the ability of an antibody to eliminate, or significantly reduce, the activity of a target antigen. Accordingly, a "neutralizing" anti-Angiopoietin-2 antibody is capable of eliminating or significantly reducing the activity of Angiopoietin-2. A neutralizing Angiopoietin-2 antibody may, for example, act by blocking the binding of Angiopoietin-2 to its receptor Tie-2. By blocking this binding, the Tie-2 mediated signal transduction is significantly, or completely, eliminated. Ideally, a neutralizing antibody against Angiopoietin-2 inhibits angiogenesis.

The term "polypeptide" is used herein as a generic term to refer to native protein, fragments, or analogs of a polypeptide sequence. Hence, native protein, fragments, and analogs are species of the polypeptide genus. Preferred polypeptides in accordance with the invention comprise the human heavy chain immunoglobulin molecules and the human kappa light chain immunoglobulin molecules, as well as antibody molecules formed by combinations comprising the heavy chain immunoglobulin molecules with light chain immunoglobulin molecules, such as the kappa or lambda light chain immunoglobulin molecules, and vice versa, as well as fragments and analogs thereof. Preferred polypeptides in accordance with the invention may also comprise solely the human heavy chain immunoglobulin molecules or fragments thereof.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory or otherwise is naturally-occurring.

The term "polynucleotide" as referred to herein means a polymeric form of nucleotides of at least 10 bases in length, either ribonucleotides or deoxynucleotides or a modified form of either type of nucleotide, or RNA-DNA hetero-duplexes. The term includes single and double stranded forms of DNA.

The term "oligonucleotide" referred to herein includes naturally occurring, and modified nucleotides linked together by naturally occurring, and non-naturally occurring linkages. Oligonucleotides are a polynucleotide subset generally comprising a length of 200 bases or fewer. Preferably, oligonucleotides are 10 to 60 bases in length and most preferably 12, 13, 14, 15, 16, 17, 18, 19, or 20 to 40 bases in length. Oligonucleotides are usually single stranded, e.g. for probes; although oligonucleotides may be double stranded, e.g. for use in the construction of a gene mutant. Oligonucleotides can be either sense or antisense oligonucleotides.

Two amino acid sequences are "homologous" if there is a partial or complete identity between their sequences. For example, 85% homology means that 85% of the amino acids are identical when the two sequences are aligned for maximum matching. Gaps (in either of the two sequences being matched) are allowed in maximizing matching; gap lengths of 5 or less are preferred with 2 or less being more preferred. Alternatively and preferably, two protein sequences (or polypeptide sequences derived from them of at least about 30 amino acids in length) are homologous, as this term is used herein, if they have an alignment score of at more than 5 (in standard deviation units) using the program ALIGN with the mutation data matrix and a gap penalty of 6 or greater. See Dayhoff, M.O., in Atlas of Protein Sequence and Structure, pp. 101-110 (Volume 5, National Biomedical Research Foundation (1972)) and Supplement 2 to this volume, pp. 1-10. The two sequences or parts thereof are more preferably homologous if their amino acids are greater than or equal to 50% identical when optimally aligned using the ALIGN program. It should be appreciated that there can be differing regions of homology within two orthologous sequences. For example, the functional sites of mouse and human orthologues may have a higher degree of homology than non-functional regions.

As used herein, the twenty conventional amino acids and their abbreviations follow conventional usage. See Immunology - A Synthesis (2nd Edition, E.S. Golub and D.R. Gren, Eds., Sinauer Associates, Sunderland, Mass. (1991)), which is incorporated herein by reference. Stereoisomers (e.g., D-amino acids) of the twenty conventional amino acids, unnatural amino acids such as α-, α-disubstituted amino acids, N-alkyl amino acids, lactic acid, and other unconventional amino acids may also be suitable components for polypeptides of the present invention. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and imino acids (e.g., 4-hydroxyproline). In the polypeptide notation used herein, the left-hand direction is the amino terminal direction and the right-hand direction is the carboxy-terminal direction, in accordance with standard usage and convention.

Similarly, unless specified otherwise, the left-hand end of single-stranded polynucleotide sequences is the 5' end; the left-hand direction of double-stranded polynucleotide sequences is referred to as the 5' direction. The direction of 5' to 3' addition of nascent RNA transcripts is referred to as the transcription direction; sequence regions on the DNA strand having the same sequence as the RNA and which are 5' to the 5' end of the RNA transcript are referred to as "upstream sequences"; sequence regions on the DNA strand having the same sequence as the RNA and which are 3' to the 3' end of the RNA transcript are referred to as "downstream sequences".

As discussed herein, minor variations in the amino acid sequences of antibodies or immunoglobulin molecules are contemplated as being encompassed by the present invention, providing that the variations in the amino acid sequence maintain at least 75%, more preferably at least 80%, 90%, 95%, and most preferably 99% sequence identity to the antibodies or immunoglobulin molecules described herein. In particular, conservative amino acid replacements are contemplated. Conservative replacements are those that take place within a family of amino acids that have related side chains. Genetically encoded amino acids are generally divided into families: (1) acidic=aspartate, glutamate; (2) basic=lysine, arginine, histidine; (3) non-polar=alanine, valine, leucine, isoleucine, proline, phenylalanine, methionine, tryptophan; and (4) uncharged polar=glycine, asparagine, glutamine, cysteine, serine, threonine, tyrosine. More preferred families are: serine and threonine are an aliphatic-hydroxy family; asparagine and glutamine are an amide-containing family; alanine, valine, leucine and isoleucine are an aliphatic family; and phenylalanine, tryptophan, and tyrosine are an aromatic family. For example, it is reasonable to expect that an isolated replacement of a leucine with an isoleucine or valine, an aspartate with a glutamate, a threonine with a serine, or a similar replacement of an amino acid with a structurally related amino acid will not have a major effect on the binding function or properties of the resulting molecule, especially if the replacement does not involve an amino acid within a framework site. Whether an amino acid change results in a functional peptide can readily be determined by assaying the specific activity of the polypeptide derivative. Assays are described in detail herein. Fragments or analogs of antibodies or immunoglobulin molecules can be readily prepared by those of ordinary skill in the art. Preferred amino- and carboxy-termini of fragments or analogs occur near boundaries of functional domains. Structural and functional domains can be identified by comparison of the nucleotide and/or amino acid sequence data to public or proprietary sequence databases. Preferably, computerized comparison methods are used to identify sequence motifs or predicted protein conformation domains that occur in other proteins of known structure and/or function. Methods to identify protein sequences that fold into a known three-dimensional structure are known. Bowie et al. Science 253:164 (1991). Thus, the foregoing examples demonstrate that those of skill in the art can recognize sequence motifs and structural conformations that may be used to define structural and functional domains in accordance with the antibodies described herein.

Preferred amino acid substitutions are those which: (1) reduce susceptibility to proteolysis, (2) reduce susceptibility to oxidation, (3) alter binding affinity for forming protein complexes, (4) alter binding affinities, and (4) confer or modify other physicochemical or functional properties of such analogs. Analogs can include various muteins of a sequence other than the naturally-occurring peptide sequence. For example, single or multiple amino acid substitutions (preferably conservative amino acid substitutions) may be made in the naturally-occurring sequence (preferably in the portion of the polypeptide outside the domain(s) forming intermolecular contacts. A conservative amino acid substitution should not substantially change the structural characteristics of the parent sequence (e.g., a replacement amino acid should not tend to break a helix that occurs in the parent sequence, or disrupt other types of secondary structure that characterizes the parent sequence). Examples of art-recognized polypeptide secondary and tertiary structures are described in Proteins, Structures and Molecular Principles (Creighton, Ed., W. H. Freeman and Company, New York (1984)); Introduction to Protein Structure (C. Branden and J. Tooze, eds., Garland Publishing, New York, N.Y. (1991)); and Thornton et al. Nature 354:105 (1991), which are each incorporated herein by reference.

The term "polypeptide fragment" as used herein refers to a polypeptide that has an amino-terminal and/or carboxy-terminal deletion, but where the remaining amino acid sequence is identical to the corresponding positions in the naturally-occurring sequence deduced, for example, from a full-length cDNA sequence. Fragments typically are at least 5, 6, 8 or 10 amino acids long, preferably at least 14 amino acids long, more preferably at least 20 amino acids long, usually at least 50 amino acids long, and even more preferably at least 70 amino acids long. The term "analog" as used herein refers to polypeptides which are comprised of a segment of at least 25 amino acids that has substantial identity to a portion of a deduced amino acid sequence and which has at least one of the following properties: (1) specific binding to a Angiopoietin-2, under suitable binding conditions, (2) ability to block appropriate Angiopoietin-2 binding, or (3) ability to inhibit Angiopoietin-2 activity. Typically, polypeptide analogs comprise a conservative amino acid substitution (or addition or deletion) with respect to the naturally-occurring sequence. Analogs typically are at least 20 amino acids long, preferably at least 50 amino acids long or longer, and can often be as long as a full-length naturally-occurring polypeptide.

Peptide analogs are commonly used in the pharmaceutical industry as non-peptide drugs with properties analogous to those of the template peptide. These types of non-peptide compound are termed "peptide mimetics" or "peptidomimetics". Fauchere, J. Adv. Drug Res. 15:29 (1986); Veber and Freidinger TINS p.392 (1985); and Evans et al. J. Med. Chem. 30:1229 (1987), which are incorporated herein by reference. Such compounds are often developed with the aid of computerized molecular modeling. Peptide mimetics that are structurally similar to therapeutically useful peptides may be used to produce an equivalent therapeutic or prophylactic effect. Generally, peptidomimetics are structurally similar to a paradigm polypeptide (i.e., a polypeptide that has a biochemical property or pharmacological activity), such as human antibody, but have one or more peptide linkages optionally replaced by a linkage selected from the group consisting of: --CH₂NH--, --CH₂S--, --CH₂-CH₂--,-CH=CH--(cis and trans), --COCH₂--, --CH(OH)CH₂--, and --CH₂SO--, by methods well known in the art. Systematic substitution of one or more amino acids of a consensus sequence with a D-amino acid of the same type (e.g., D-lysine in place of L-lysine) may be used to generate more stable peptides. In addition, constrained peptides comprising a consensus sequence or a substantially identical consensus sequence variation may be generated by methods known in the art (Rizo and Gierasch Ann. Rev. Biochem. 61:387 (1992), incorporated herein by reference); for example, by adding internal cysteine residues capable of forming intramolecular disulfide bridges which cyclize the peptide.

"Binding fragments" of an antibody are produced by recombinant DNA techniques, or by enzymatic or chemical cleavage of intact antibodies. Binding fragments include Fab, Fab', F(ab')₂, Fv, and single-chain antibodies. An antibody other than a "bispecific" or "bifunctional" antibody is understood to have each of its binding sites identical. An antibody substantially inhibits adhesion of a receptor to a counterreceptor when an excess of antibody reduces the quantity of receptor bound to counterreceptor by at least about 20%, 40%, 60% or 80%, and more usually greater than about 85% (as measured in an *in vitro* competitive binding assay).

The term "epitope" includes any protein determinant capable of specific binding to an immunoglobulin or T-cell receptor. Epitopic determinants usually consist of chemically active surface groupings of molecules such as amino acids or sugar side chains and may, but not always, have specific three-dimensional structural characteristics, as well as specific charge characteristics. An antibody is said to specifically bind an epitope when the dissociation constant is ≤1 µM, preferably ≤ 100 nM and most preferably ≤ 10 nM.

The term "agent" is used herein to denote a chemical compound, a mixture of chemical compounds, a biological macromolecule, or an extract made from biological materials.

"Active" or "activity" in regard to an Angiopoietin-1 or an Angiopoietin-2 polypeptide refers to a portion of the polypeptide that has a biological or an immunological activity as per the native polypeptide. "Biological" when used herein refers to a biological function that results from the activity of the native polypeptide. For example, a preferred Angiopoietin-2 biological activity includes Angiopoietin-2 induced angiogenesis.

"Mammal" refers to all mammals, but preferably the mammal is human.

Digestion of antibodies with the enzyme, papain, results in two identical antigen-binding fragments, known also as "Fab" fragments, and a "Fc" fragment, having no antigen-binding activity but having the ability to crystallize. Digestion of antibodies with the enzyme, pepsin, results in the a F(ab')₂ fragment in which the two arms of the antibody molecule remain linked and comprise two-antigen binding sites. The F(ab')₂ fragment has the ability to crosslink antigen.

"Fv" when used herein refers to the minimum fragment of an antibody that retains both antigen-recognition and antigen-binding sites.

"Fab" when used herein refers to a fragment of an antibody that comprises the constant domain of the light chain and the CH1 domain of the heavy chain.

The term "mAb" refers to monoclonal antibody.

"Liposome" when used herein refers to a small vesicle that may be useful for delivery of drugs that may include the Angiopoietin-2 polypeptide of the invention or antibodies to such an Angiopoietin-2 polypeptide to a mammal.

"Label" or "labeled" as used herein refers to the addition of a detectable moiety to a polypeptide, for example, a radiolabel, fluorescent label, enzymatic label chemiluminescent labeled or a biotinyl group. Radioisotopes or radionuclides may include ³H, ¹⁴C, ¹⁵N, ³⁵S, ⁹⁰Y, ⁹⁹Tc, ¹¹¹In, ¹²⁵I, ¹³¹I, fluorescent labels may include rhodamine, lanthanide phosphors or FITC and enzymatic labels may include horseradish peroxidase, β-galactosidase, luciferase, alkaline phosphatase.

The term "pharmaceutical agent or drug" as used herein refers to a chemical compound, combination or composition capable of inducing a desired therapeutic effect when properly administered to a patient. Other chemistry terms herein are used according to conventional usage in the art, as exemplified by The McGraw-Hill Dictionary of Chemical Terms (Parker, S., Ed., McGraw-Hill, San Francisco (1985)), (incorporated herein by reference).

The term "patient" includes human and veterinary subjects.

The invention will now be illustrated by the following non-limiting examples, which are provided for illustrative purposes only and are not to be construed as limiting upon the teachings herein, in which:
Figure 1a. Shows combination efficacy following treatment with mAb 3.19.3 and VTKI (VEGF Tyrosine Kinase Inhibitor (-4-(4-fluoro-2-methylindol-5-yloxy)-6-methoxy-7-(3-piperidinopropoxy)quinazoline)) in mice bearing A431 xenograft tumours.
Figure 1b. Shows effects on host body weight changes following combination treatment with mAb 3.19.3 and VTKI (VEGF Tyrosine Kinase Inhibitor (-4-(4-fluoro-2-methylindol-5-yloxy)-6-methoxy-7-(3-piperidinopropoxy)quinazoline)) in mice bearing A431 xenograft tumours.
Figure 2a. Shows combination efficacy following treatment with mAb 3.19.3 and VTKI (VEGF Tyrosine Kinase Inhibitor (-4-(4-fluoro-2-methylindol-5-yloxy)-6-methoxy-7-(3-piperidinopropoxy)quinazoline)) in mice bearing Colo205 xenograft tumours.
Figure 2b. Shows effects on host body weight changes following combination treatment with mAb 3.19.3 and VTKI (VEGF Tyrosine Kinase Inhibitor (-4-(4-fluoro-2-methylindol-5-yloxy)-6-methoxy-7-(3-piperidinopropoxy)quinazoline)) in mice bearing Colo205 xenograft tumours.
Figure 3a. Shows combination efficacy following treatment with mAb 3.19.3 and AZD2171 in mice bearing HT29 xenograft tumours.
Figure 3b. Shows effects on host body weight changes following combination treatment with mAb 3.19.3 and AZD2171 in mice bearing HT29 xenograft tumours.
Figure 4a. Shows combination efficacy following treatment with mAb 3.19.3 and Zactima™ in mice bearing LoVo xenograft tumours.
Figure 4b. Shows effects on host body weight changes following combination treatment with mAb 3.19.3 and Zactima™ in mice bearing LoVo xenograft tumours.
Figure 5a. Shows combination efficacy following treatment with mAb 3.19.3 and mAb DC101 in mice bearing SW620 colon xenograft tumours..
Figure 5b. Shows effects on host body weight changes following combination treatment with mAb 3.19.3 and mAb DC101 in mice bearing SW620 colon xenograft tumours.

### EXAMPLE 1: ANTIBODY GENERATION

### Immunisation

Recombinant human Angiopoietin-2 obtained from R&D Systems, Inc. (Minneapolis, MN Cat. No. 623-AM/CF) was used as an antigen. Monoclonal antibodies against Angiopoietin-2 were developed by sequentially immunizing XenoMouse^{®} mice (XenoMouse strains XMG2 and XMG4 (3C-1 strain), Abgenix, Inc. Fremont, CA). XenoMouse animals were immunized via footpad route for all injections. The total volume of each injection was 50 µl per mouse, 25 µl per footpad. The first injection was with 2.35 µg recombinant human Angiopoietin-2 (rhAngiopoietin-2, cat#623-AM/CF; lot #BN023202A) in pyrogen-free Dulbecco's PBS (DPBS) and admixed 1:1 v/v with 10 µg CpG (15 µl of ImmunEasy Mouse Adjuvant, catalog # 303101; lot #11553042; Qiagen) per mouse. The next 6 boosts were with 2.35 µg rhANGIOPOIETIN-2 in pyrogen-free DPBS, admixed with 25 µg of Adju-Phos (aluminum phosphate gel, Catalog # 1452-250, batch #8937, HCI Biosector) and 10 µg CpG per mouse, followed by a final boost of 2.35 µg rhAngiopoietin-2 in pyrogen-free DPBS, without adjuvant. The XenoMouse mice were immunized on days 0, 3, 6, 10, 13, 17, 20, and 24 for this protocol and fusions were performed on day 29.

### Selection of Animals for Harvest by Titer

Anti-Angiopoietin-2 antibody titers in the serum from immunized XenoMouse mice were determined by ELISA. Briefly, recombinant Angiopoietin-2 (1 µg/ml) was coated onto Costar Labcoat Universal Binding Polystyrene 96-well plates (Coming, Acton, MA) overnight at four degrees in Antigen Coating Buffer (0.1 M Carbonate Buffer, pH 9.6 NaHCO₃ 8.4 g/L). The next day, the plates were washed 3 times with washing buffer (0.05% Tween 20 in 1x PBS) using a Biotek plate washer. The plates were then blocked with 200 µl/well blocking buffer (0.5% BSA, 0.1% Tween 20, 0.01% Thimerosal in 1x PBS) and incubated at room temperature for 1 h. After the one-hour blocking, the plates were washed 3 times with washing buffer using a Biotek plate washer. Sera from either Angiopoietin-2 immunized XenoMouse mice or naïve XenoMouse animals were titrated in 0.5% BSA/PBS buffer at 1:3 dilutions in duplicate from a 1:100 initial dilution. The last well was left blank. These plates were incubated at room temperature for 2 hr, and the plates were then washed 3 times with washing buffer using a Biotek plate washer. A goat anti-human IgG Fc-specific horseradish peroxidase (HRP, Pierce, Rockford, IL) conjugated antibody was added at a final concentration of 1 µg/ml and incubated for 1 hour at room temperature. Then the plates were washed 3 times with washing buffer using a Biotek plate washer.

After washing, the plates were developed with the addition of TMB chromogenic substrate (BioFx BSTP-0100-01) for 10-20 min or until negative control wells start to show color. Then the ELISA was stopped by the addition of Stop Solution (650 nM Stop reagent for TMB (BioFx BSTP-0100-01), reconstituted with 100 ml H₂O per bottle). The specific titer of each XenoMouse animal was determined from the optical density at 650nm.

### Recovery of lymphocytes, B-cell isolations, fusions and generation of hybridomas

Immunized mice were sacrificed by cervical dislocation, and the draining lymph nodes harvested and pooled from each cohort. The lymphoid cells were dissociated by grinding in DMEM to release the cells from the tissues and the cells were suspended in DMEM. The cells were counted, and 0.9 ml DMEM per 100 million lymphocytes added to the cell pellet to resuspend the cells gently but completely. Using 100 µl of CD90+ magnetic beads per 100 million cells, the cells were labeled by incubating the cells with the magnetic beads at 4°C for 15 minutes. The magnetically labeled cell suspension containing up to 10⁸ positive cells (or up to 2x10⁹ total cells) was loaded onto a LS+ column and the column washed with DMEM. The total effluent was collected as the CD90-negative fraction (most of these cells were expected to be B cells).

The fusion was performed by mixing washed enriched B cells from above and nonsecretory myeloma P3X63Ag8.653 cells purchased from ATCC, cat.# CRL 1580 (Kearney et al, J. Immunol. 123, 1979, 1548-1550) at a ratio of 1:1. The cell mixture was gently pelleted by centrifugation at 800 x g. After complete removal of the supernatant, the cells were treated with 2-4 mL of Pronase solution (CalBiochem, cat. # 53702; 0.5 mg/ml in PBS) for no more than 2 minutes. Then 3-5 ml of FBS was added to stop the enzyme activity and the suspension was adjusted to 40 ml total volume using electro cell fusion solution, (ECFS, 0.3M Sucrose, Sigma, Cat# S7903, 0.1mM Magnesium Acetate, Sigma, Cat# M2545, 0.1mM Calcium Acetate, Sigma, Cat# C4705). The supernatant was removed after centrifugation and the cells were resuspended in 40 ml ECFS. This wash step was repeated and the cells again were resuspended in ECFS to a concentration of 2x10⁶ cells/ml.

Electro-cell fusion was performed using a fusion generator (model ECM2001, Genetronic, Inc., San Diego, CA). The fusion chamber size used was 2.0 ml, using the following instrument settings:
Alignment condition: voltage: 50 V, time: 50 sec.
Membrane breaking at: voltage: 3000 V, time: 30 µsec
Post-fusion holding time: 3 sec

After ECF, the cell suspensions were carefully removed from the fusion chamber under sterile conditions and transferred into a sterile tube containing the same volume of Hybridoma Culture Medium (DMEM, JRH Biosciences), 15 % FBS (Hyclone), supplemented with L-glutamine, pen/strep, OPI (oxaloacetate, pyruvate, bovine insulin) (all from Sigma) and IL-6 (Boehringer Mannheim). The cells were incubated for 15-30 minutes at 37°C, and then centrifuged at 400 x g (1000 rpm) for five minutes. The cells were gently resuspended in a small volume of Hybridoma Selection Medium (Hybridoma Culture Medium supplemented with 0.5x HA (Sigma, cat. # A9666)), and the volume adjusted appropriately with more Hybridoma Selection Medium, based on a final plating of 5x10⁶ B cells total per 96-well plate and 200 µl per well. The cells were mixed gently and pipetted into 96-well plates and allowed to grow. On day 7 or 10, one-half the medium was removed, and the cells re-fed with Hybridoma Selection Medium.

### Selection of candidate antibodies by Elisa

After 14 days of culture, hybridoma supernatants were screened for Angiopoietin-2-specific monoclonal antibodies. The ELISA plates (Fisher, Cat. No. 12-565-136) were coated with 50 µl/well of human Angiopoietin-2 (2 µg/ml) in Coating Buffer (0.1 M Carbonate Buffer, pH 9.6, NaHCO₃ 8.4 g/L), then incubated at 4°C overnight. After incubation, the plates were washed with Washing Buffer (0.05% Tween 20 in PBS) 3 times. 200 µl/well Blocking Buffer (0.5% BSA, 0.1% Tween 20, 0.01% Thimerosal in 1x PBS) were added and the plates incubated at room temperature for 1 hour. After incubation, the plates were washed with Washing Buffer three times. 50 µl/well of hybridoma supernatants, and positive and negative controls were added and the plates incubated at room temperature for 2 hours. The positive control used throughout was serum from the Angiopoietin-2 immunized XenoMouse mouse, XMG2 Angiopoietin-2 Group 1, footpad (fp) N160-7, and the negative control was serum from the KLH-immunized XenoMouse mouse, XMG2 KLH Group 1, footpad (fp) L627-6.

After incubation, the plates were washed three times with Washing Buffer. 100 µl/well of detection antibody goat anti-huIgGFc-HRP (Caltag, Cat. No. H10507) was added and the plates incubated at room temperature for 1 hour. In the secondary screen, the positives in first screening were screened in two sets, one for hIgG detection and the other for human Ig kappa light chain detection (goat anti-hIg kappa-HRP (Southern Biotechnology, Cat. No. 2060-05) in order to demonstrate fully human composition for both IgG and Ig kappa. After incubation, the plates were washed three times with Washing Buffer. 100 µl/well of TMB (BioFX Lab. Cat. No. TMSK-0100-01) were added and the plates allowed to develop for about 10 minutes (until negative control wells barely started to show color). 50 µl/well stop solution (TMB Stop Solution, (BioFX Lab. Cat. No. STPR-0100-01) was then added and the plates read on an ELISA plate reader at 450nm. There were 185 fully human IgG kappa antibodies against Angiopoietin-2.

All antibodies that bound in the ELISA assay can be counter screened for binding to Angiopoietin-1 by ELISA in order to identify those that cross-react with Angiopoietin-1. The ELISA plates (Fisher, Cat. No. 12-565-136) were coated with 50 µl/well of recombinant Angiopoietin-1 (2 µg/ml, obtained from R&D Systems, Cat. # 293-AN-025/CF) in Coating Buffer (0.1 M Carbonate Buffer, pH 9.6, NaHCO₃ 8.4 g/L), then incubated at 4°C overnight.

### Antibody identification number and SEQ ID number

Table 1 below reports the identification number of the anti-Angiopoietin-2 antibody with the SEQ ID number of the corresponding heavy chain and light chain genes.

**Table 1**

| **mAb ID No.:** | **Sequence** | **SEQ ID NO:** |
|---|---|---|
| **3.3.2** | Nucleotide sequence encoding the variable region of the heavy chain | 1 |
| | Amino acid sequence encoding the variable region of the heavy chain | 2 |
| | Nucleotide sequence encoding the variable region of the light chain | 3 |
| | Amino acid sequence encoding the variable region of the light chain | 4 |
| **3.19.3** | Nucleotide sequence encoding the variable region of the heavy chain | 5 |
| | Amino acid sequence encoding the variable region of the heavy chain | 6 |
| | Nucleotide sequence encoding the variable region of the light chain | 7 |
| | Amino acid sequence encoding the variable region of the light chain | 8 |
| **3.31.2** | Nucleotide sequence encoding the variable region of the heavy chain | 9 |
| | Amino acid sequence encoding the variable region of the heavy chain | 10 |
| | Nucleotide sequence encoding the variable region of the light chain | 11 |
| | Amino acid sequence encoding the variable region of the light chain | 12 |
| **5.16.3** | Nucleotide sequence encoding the variable region of the heavy chain | 13 |
| | Amino acid sequence encoding the variable region of the heavy chain | 14 |
| | Nucleotide sequence encoding the variable region of the light chain | 15 |
| | Amino acid sequence encoding the variable region of the light chain | 16 |
| **5.28.1** | Nucleotide sequence encoding the variable region of the heavy chain | 17 |
| | Amino acid sequence encoding the variable region of the heavy chain | 18 |
| | Nucleotide sequence encoding the variable region of the light chain | 19 |
| | Amino acid sequence encoding the variable region of the light chain | 20 |
| **5.78.3** | Nucleotide sequence encoding the variable region of the heavy chain | 21 |
| | Amino acid sequence encoding the variable region of the heavy chain | 22 |
| | Nucleotide sequence encoding the variable region of the light chain | 23 |
| | Amino acid sequence encoding the variable region of the light chain | 24 |
| **5.86.1** | Nucleotide sequence encoding the variable region of the heavy chain | 25 |
| | Amino acid sequence encoding the variable region of the heavy chain | 26 |
| | Nucleotide sequence encoding the variable region of the light chain | 27 |
| | Amino acid sequence encoding the variable region of the light chain | 28 |
| **5.88.3** | Nucleotide sequence encoding the variable region of the heavy chain | 29 |
| | Amino acid sequence encoding the variable region of the heavy chain | 30 |
| | Nucleotide sequence encoding the variable region of the light chain | 31 |
| | Amino acid sequence encoding the variable region of the light chain | 32 |
| **5.101.1** | Nucleotide sequence encoding the variable region of the heavy chain | 33 |
| | Amino acid sequence encoding the variable region of the heavy chain | 34 |
| | Nucleotide sequence encoding the variable region of the light chain | 35 |
| | Amino acid sequence encoding the variable region of the light chain | 36 |
| **5.103.1** | Nucleotide sequence encoding the variable region of the heavy chain | 37 |
| | Amino acid sequence encoding the variable region of the heavy chain | 38 |
| | Nucleotide sequence encoding the variable region of the light chain | 39 |
| | Amino acid sequence encoding the variable region of the light chain | 40 |

### Inhibition of Angiopoietin-2 binding to Tie-2

As discussed above, Angiopoietin-2 exerts its biological effect by binding to the Tie-2 receptor. Monoclonal antibodies that inhibited Angiopoietin-2/Tie-2 binding were identified by a competitive binding assay using a modified ELISA. The mAb used were products of micro-purification from 50 ml of exhaustive supernatants of the hybridoma pools that were specific for Angiopoietin-2 (see above). 96-well Nunc Immplates™ were coated with 100 µl of recombinant human Tie-2/Fc fusion protein (R&D Systems, Inc., Cat. No. 313-TI-100) at 4 µg/ml by incubating overnight at 4°C. The plates were washed four times using Phosphate Buffer Saline (PBS) with a Skan™ Washer 300 station (SKATRON). The wells were blocked by 100 µl of ABX-blocking buffer (0.5% BSA, 0.1%Tween, 0.01% Thimerosal in PBS) for 1 hour.

Biotinylated recombinant human Angiopoietin-2 (R&D Systems, Inc. Cat. No. BT623) at 100 ng/ml was added in each well with or without the anti Angiopoietin-2 mAb at 100 µg/ml. The plates were incubated at room temperature for two hours before the unbound molecules were washed off. Bound biotinylated Angiopoietin-2 was then detected using 100 µl/well of Streptavidin-HRP conjugate at 1:200 by incubating at room temperature for half an hour. After washing twice, the bound Streptavidin was detected by HRP substrate (R&D Systems, Cat. No. DY998). The plates were incubated for 30 minutes before 450 stop solution (100 µl/well, BioFX, Cat# BSTP-0100-01) was added to terminate the reaction. The light absorbance at 450 nm was determined by a Spectramax Plus reader.

Soluble recombinant Tie-2/Fc fusion protein at 10-fold molar excess to Angiopoietin-2 was used as a positive control. At this concentration, Tie-2/Fc inhibited binding of Angiopoietin-2 to immobilized Tie-2 by 80%. With this as an arbitrary criterion, 74 out of 175 Angiopoietin-2 binding mAbs showed inhibitory activity.

Each hybridoma was cloned using a limited dilution method by following standard procedures. Three sister clones were collected from each hybridoma. For each clone, the supernatant was tested using ELISA binding to human Angiopoietin-2 and counter binding to Angiopoietin-1, as described above, to ensure that each antibody was only specific for Angiopoietin-2. Concentrations of IgG in the exhaustive supernatants were determined, and one clone with the highest yield among the three sister clones from each hybridoma was selected for IgG purification. 0.5 to 1 mg of IgG was purified from each supernatant for further characterization.

To quantitate the inhibitory activities of the mAb on Angiopoietin-2 binding to Tie-2, the titer was determined for purified mAbs from the top candidates using a competitive binding assay. Each concentration of the mAb was tested in duplicate. The concentration-response relationship was found by curve fitting using Graphpad Prism™ graphic software (non-linear, Sigmoid curve). The maximal inhibition (efficacy) and IC₅₀ (potency) were calculated by the software. Ten monoclonal antibodies that exhibited both high efficacy and potency were selected; the efficacy and potency of these mAbs are shown in Table 2.

**Table 2. Efficacy and Potency anti-Angiopoietin-1/ Angiopoietin-2 mAb**

| **Clone** | **Efficacy*** | **EC50 (µg/ml)** |
|---|---|---|
| 3.31.2 | 0.3751 | 0.04169 |
| 5.16.3 | 0.3279 | 0.08532 |
| 5.86.1 | 0.3844 | 0.1331 |
| 5.88.3 | 0.4032 | 0.1557 |
| 3.3.2 | 0.3881 | 0.1684 |
| 5.103.1 | 0.2317 | 0.3643 |
| 5.101.1 | 0.3639 | 0.3762 |
| 3.19.3 | 0.3945 | 0.7976 |
| 5.28.1 | 0.3892 | 2.698 |
| 5.78.3 | 0.2621 | 5.969 |

| | | |
|---|---|---|
| * Efficacy is expressed as the ratio of bound Angiopoietin-2 with mAb (30 µg/ml) versus without mAb. | | |

The cross-reactivity of mAb 3.19.3 to Angiopoietin-1 was then investigated by measuring the affinity of the mAb to Angiopoietin-1.

### Determination of anti-Angiopoietin-1 antibody affinity using Biacore analysis

The cross-reactivity of the antibody to Angiopoietin-1 was further investigated by measuring the affinity of the mAbs to Angiopoietin-1. Instead of immobilizing Angiopoietin-1, as described in ELISA-based counter-binding, the mAbs were immobilized to the CM5 Biacore chips, and Angiopoietin-1 in solution was injected for the determination of the on-rate and off-rate. Six mAbs; 3.3.2, 3.31.2, 5.16.3. 5.86.1, 5.88.3 and 3.19.3 were tested.

### Medium Resolution Screen

Label-free surface plasmon resonance (SPR), or Biacore 2000 instrumentation, was utilized to measure antibody affinity to Angiopoietin-1. For this purpose, a high-density goat α-human antibody surface over a CM5 Biacore chip was prepared using routine amine coupling. For developmental experiments, purified mAbs (clones 3.3.2, 3.31.2, 5.16.3. 5.86.1, 5.88.3 and 3.19.3) were diluted to approximately 2.5-3.5 µg/ml in HBS-P running buffer containing 100 µg/ml BSA. The capture level for each mAb was approximately 150 RU. A 5-minute wash followed each capture cycle to stabilize the mAb baseline.
A single Angiopoietin-1 sample diluted to 87.4 nM in the running buffer was injected for one minute over all capture surfaces. Angiopoietin-1 was found to bind to mAb 3.19.3. This experiment was repeated by increasing the mAb capture levels to well over 500-600 RU and injecting 380 nM Angiopoietin-1 for one minute. Angiopoietin-1 was again found to bind mAb 3.19.3.

### EXAMPLE 2: COMBINATION STUDIES

The activity of mAb 3.19.3 in combination with small molecule VEGF tyrosine kinase inhibitors has been evaluated.

### Determination of the therapeutic efficacy of mAb 3.19.3 in combination with 4-(4-fluoro-2-methylindol-5-yloxy)-6-methoxy-7-(3-piperidinopropoxy)quinazoline in A431 and Colo205 xenograft models

The anti-tumor activity of monoclonal antibody 3.19.3 in combination with the VTKI 4-(4-fluoro-2-methylindol-5-yloxy)-6-methoxy-7-(3-piperidinopropoxy)quinazoline was evaluated in a xenograft model of human skin epidermoid carcinoma (Study A) and in a model of colorectal cancer (Study B) by using the A431 and Colo205 cell lines respectively.

A431 and Colo205 cells were cultured in flasks as routine until the cells reached sub-confluence. Immunodeficient 6-8 week old female mice (Ncr/nu/nu) were used. The cells were harvested and suspended in Matrigel. A cell suspension containing 1 to 5 x 10⁶ cells was injected subcutaneously into the flank of the mice. The mice were randomized into different groups, each containing 10-15 mice. When the tumour volume reached 200mm³, the mice were randomized in each groups and the treatments were initiated. mAb 3.19.3 10mg/kg in saline was injected intraperitoneally, twice per week for 2 weeks. 4-(4-fluoro-2-methylindol-5-yloxy)-6-methoxy-7-(3-piperidinopropoxy)quinazoline was treated peroral daily at doses ranging from 1.5 to 6mg/kg in water containing 1% Tween80. The dimensions of each tumor were measured twice per week. The volume of the tumor was calculated as: Volume = Length x (Width)² x 0.5 (cm³).

### Study A: Determination of the therapeutic efficacy of mAb 3.19.3 in combination with 4-(4-fluoro-2-methylindol-5-yloxy)-6-methoxy-7-(3-piperidinopropoxy)quinazoline in A431 human tumour xenografts

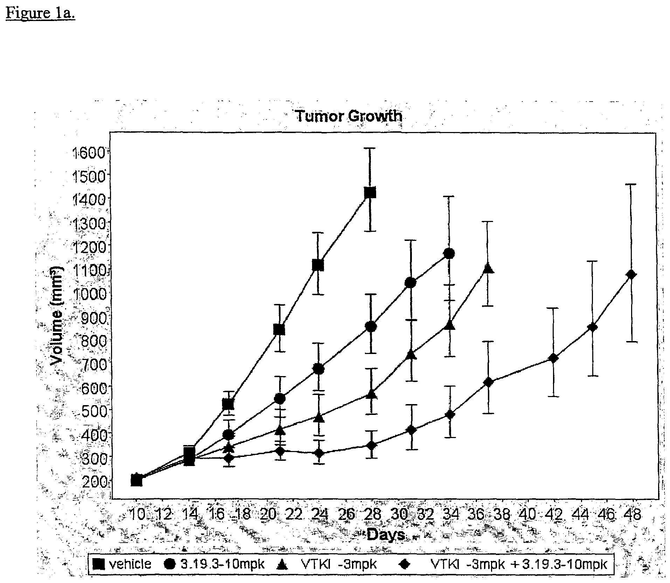

Results of the A431 combination xenograft efficacy study are shown in Figure 1a, which illustrates that the combination yields significantly greater activity than either single agent alone. The % tumor growth inhibition achieved is as follows:
3.19.3 (10mg/kg 2xwk) = 46%; (p<0.01)
4-(4-fluoro-2-methylindol-5-yloxy)-6-methoxy-7-(3-piperidinopropoxy)quinazoline (3mg/kg/day) = 69%; (p<0.001)
Combination 3.19.3 + 4-(4-fluoro-2-methylindol-5-yloxy)-6-methoxy-7-(3-piperidinopropoxy)quinazoline = 89% inhibition (p<0.001 for combination vs. single agent).

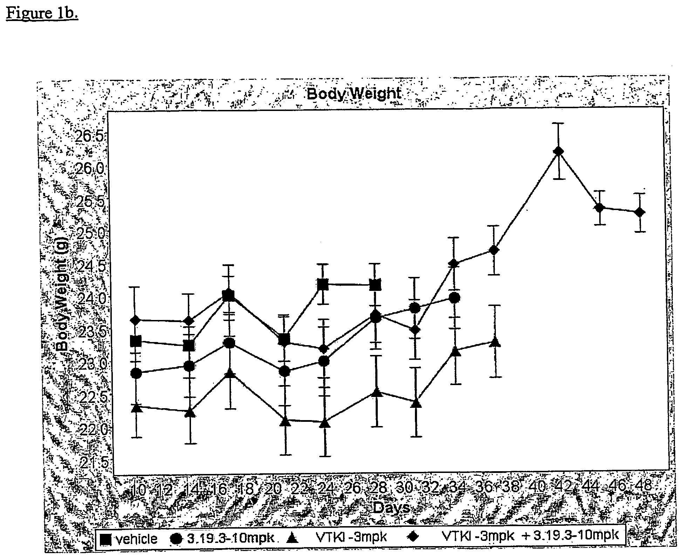

No additional toxicity was observed with the combinations as compared to single-agent treatment alone as determined by changes in body weights (Figure 1b). These results demonstrate that combination treatment with anti-Ang2 mAb 3.19.3 and the VTKI 4-(4-fluoro-2-methylindol-5-yloxy)-6-methoxy-7-(3-piperidinopropoxy)quinazoline leads to improvements in efficacy without additive toxicity in pre-clinical models and provide basis for further clinical investigation of this combination.

### Study B: Determination of the therapeutic efficacy of mAb 3.19.3 in combination with 4-(4-fluoro-2-methylindol-5-yloxy)-6-methoxy-7-(3-piperidinopropoxy)quinazoline in human Colo205 colon tumour xenografts

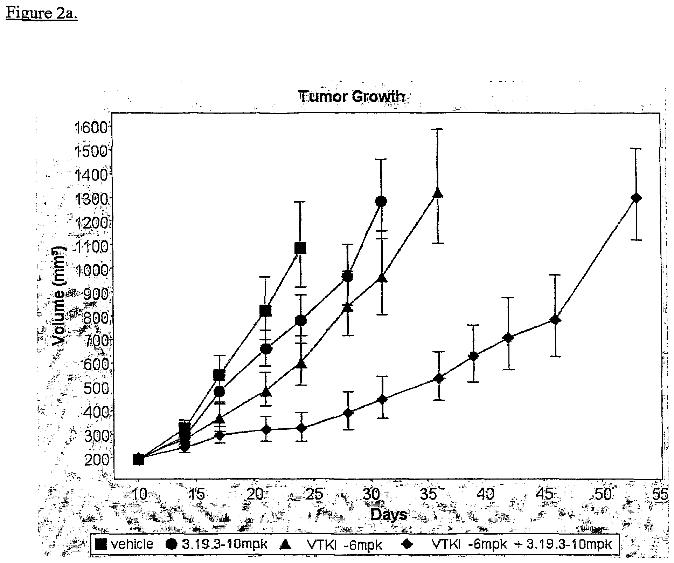

Results of the Colo205 combination xenograft efficacy study are shown in Figure 2a, which illustrates that the combination yields significantly greater activity than either single agent alone. The % inhibition achieved is as follows:
3.19.3 (10mg/kg 2xwk) = 35%; (p<0.05)
4-(4-fluoro-2-methylindol-5-yloxy)-6-methoxy-7-(3-piperidinopropoxy)quinazoline (6mg/kg/day) = 57%; (p<0.01)
Combination 3.19.3 + 4-(4-fluoro-2-methylindol-5-yloxy)-6-methoxy-7-(3-piperidinopropoxy)quinazoline = 87% inhibition (p<0.001 for combination vs. single agent).

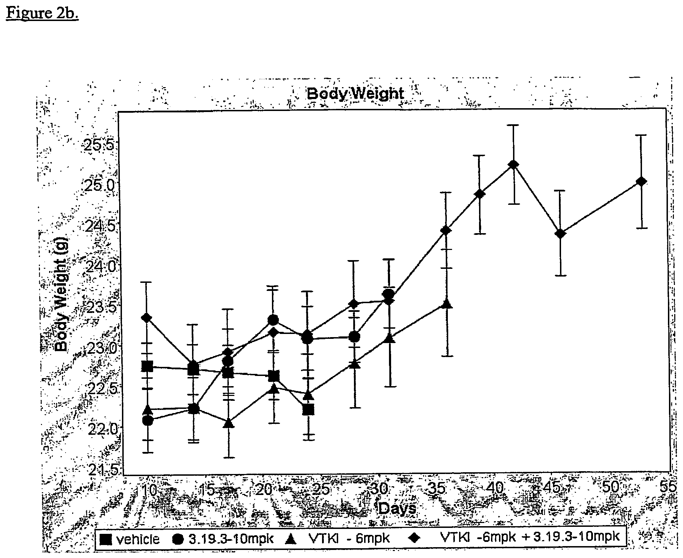

No additional toxicity was observed with the combinations as compared to single-agent treatment alone as determined by changes in body weights (Figure 2b). These results demonstrate that combination treatment with anti-Ang2 mAb 3.19.3 and the VTKI 4-(4-fluoro-2-methylindol-5-yloxy)-6-methoxy-7-(3-piperidinopropoxy)quinazoline leads to improvements in efficacy without additive toxicity in pre-clinical models and provide basis for further clinical investigation of this combination.

### Determination of the therapeutic efficacy of mAb 3.19.3 in combination with AZD2171 in human HT29 colon tumour xenografts

The efficacy of mAb 3.19.3 in combination with AZD2171 was evaluated in human HT29 xenografts. Briefly, 5x10⁶ HT29 tumour cells in 0.1 ml of serum free Roswell Park Memorial Institute (RPMI)- 1640 medium were injected subcutaneously into the flanks of 60 athymic (*nu*/*nu* genotype) mice. When tumours reached a volume of 200 to 400 mm³ (9-10 days), mice were randomized into groups (8 per group) and treatment started (day 0).

The control group (Group 1) received a daily oral (p.o.) administration of vehicle only for 28 consecutive days (day 0 - 27). Group 2 treatment consisted of a daily p.o. administration of AZD2171 alone at 1.5mg/kg/adminstration for 28 consecutive days (day 0-27). AZD2171 was prepared as a suspension in 1% polysorbate 80 (i.e. a 1% (v/v) solution of polyoxyethylene (20) sorbitan mono-oleate in deionised water). Group 3 received eight intraperitoneal (i.p) injections of mAb 3.19.3 at 10mg/kg/injection, on day 0, 3, 7, 10,14, 17, 21, and 24. Group 4 received daily p.o administration of AZD2171 at 1.5mg/kg/adminstration for 28 consecutive days (day 0 - 27) combined with eight i.p injections of mAb 3.19.3 at 10mg/kg/injection, on day 0, 3, 7, 10, 14, 17, 21 and 24. The administration volume of AZD2171 was 10.0 ml/kg (i.e. 200 µl for a 20 g mouse). The injection volume of mAb 3.19.3 was 10.0 ml/kg (i.e. 200 µl for a 20 g mouse).

**Table 3. Dosing schedule**

| **Group** | **Treatments** | **Combined drug doses (mg base/kg/inj.)** | **Adm. route** | **No. Treatments** | **No. Treatment/ day** | **Days-interval between treatment (Days)** |
|---|---|---|---|---|---|---|
| **1** | Vehicle of AZD2171 | 0.0 | p.o for AZD2171 vehicle | 28 p.o | 1 p.o | 1 for p.o |
| **2** | AZD2171 | 1.5 | p.o for AZD2171 | 28 p.o | 1 p.o | 1 for p.o |
| **3** | 3.19.3 | 10 | i.p for 3.19.3 | 8 i.p | | 3 or 4 for i.p |
| **4** | AZD2171 + 3.19.3 | 1.5 for AZD2171 | p.o for AZD2171 | 28 p.o | 1 p.o. | 1 for p.o |
| | | 10 for 3.19.3 | i.p for 3.19.3 | 8 i.p | | 3 or 4 for i.p |

Tumour volumes (mm³) were assessed at least twice weekly by bilateral Vernier caliper measurement and, taking length to be the longest diameter across the tumor and width the corresponding perpendicular, calculated using the formula (π/6) x (length x width) x √ (length x width). Growth inhibition from the start of treatment was assessed by comparison of the differences in tumor volume between control and treated groups. For all mice, the study was stopped after 28 days. For all mice, the tumours were excised and weights recorded upon termination of the study.

**Table 4. Effect of treatment on tumour growth**

| Treatment | Inhibition of Control Tumour Growth Day 28 | P value (one-tailed two-sample *t*-test) |
|---|---|---|
| AZD2171 (1.5mg/kg/day p.o, d 0 - 27) | 55 % | 0.0006 |
| 3.19.3 (10 mg/kg i.p, day 0, 3, 7, 10, 14, 17, 21 and 24) | 40 % | 0.0001 |
| AZD2171 + 3.19.3 | 81 % | <0.0001 |

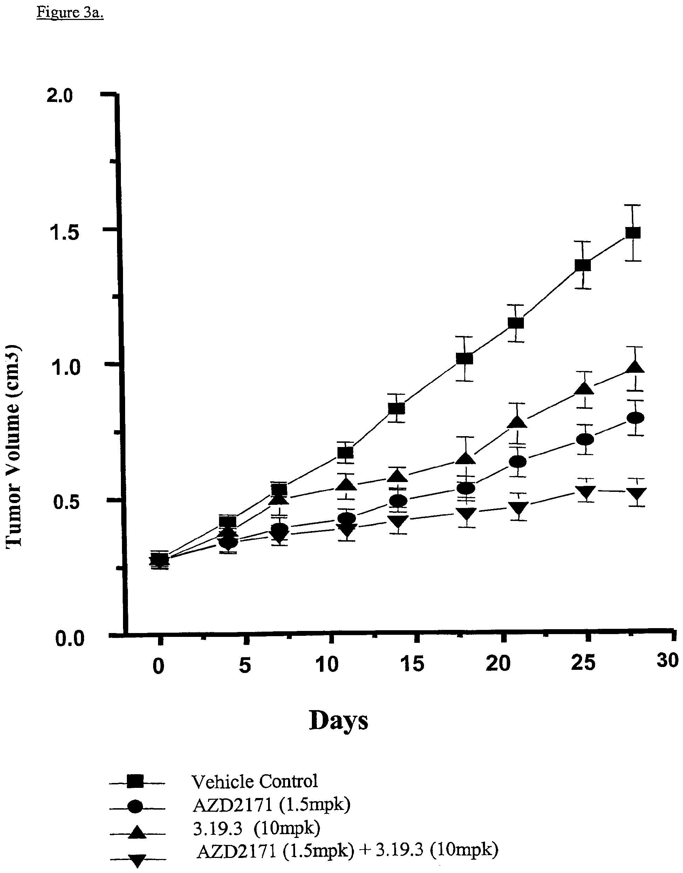

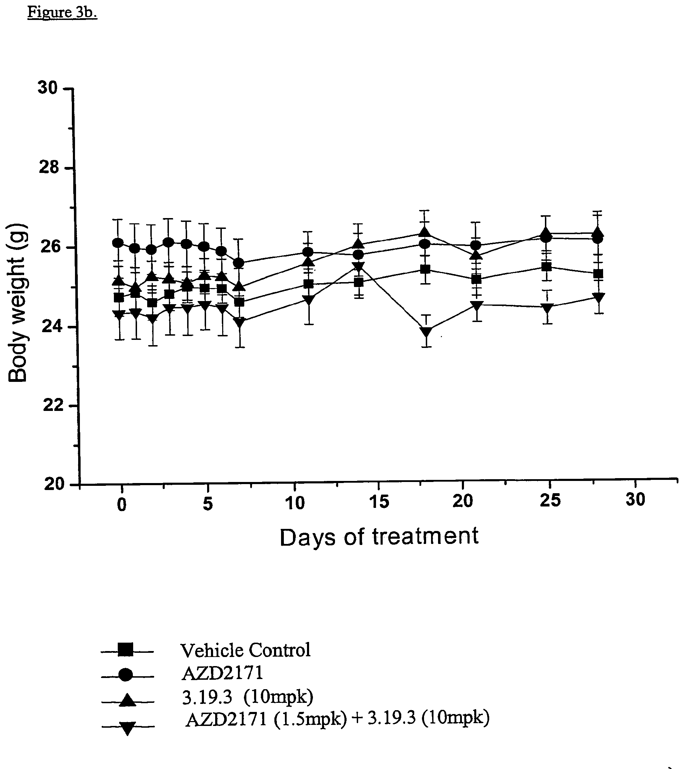

As illustrated in Figure 3a, and Table 4, the combination of mAb 3.19.3 with AZD2171 produced a significantly greater inhibition of tumour growth than 3.19.3 alone (P<0.0001 for single agent vs. combination: P value derived by one-tailed two-sample *t*-test assuming equal variance). No additional toxicity was observed with the combinations as compared to single-agent treatment alone as determined by changes in body weights (Figure 3b). These results demonstrate that combination treatment with anti-Ang2 mAb 3.19.3 and the VEGF inhibitor AZD2171 leads to improvements in efficacy without additive toxicity in pre-clinical models and provide basis for further clinical investigation of this combination.

### Determination of the therapeutic efficacy of mAb 3.19.3 in combination with Zactima™ in human LoVo colon tumour xenografts

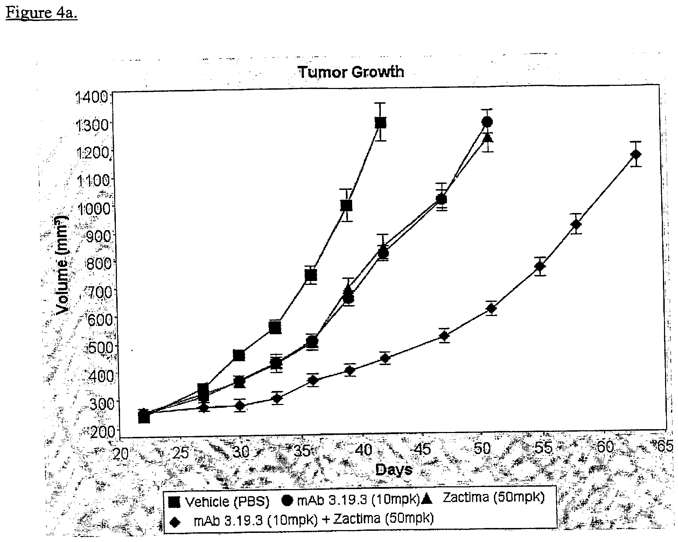

The anti-tumor activity of monoclonal antibody 3.19.3 was evaluated in the LoVo xenograft model of colorectal cancer. Briefly, LoVo cells were cultured in flasks as routine until the cells reached sub-confluence. Immunodeficient 8 week old male NCr nude mice were used. Cell suspensions containing 3 x 106 cells were injected subcutaneously into the right flank of the mice, and after the tumour volume reached 200mm³, the mice were randomized in groups and the treatments were initiated. mAb 3.19.3 10mg/kg in saline was injected intraperitoneally, twice per week for 2 weeks. Zactima™ was treated peroral daily at doses ranging from 25 to 50mg/kg in water containing 1% Tween80. The dimensions of each tumor were measured twice per week. The volume of the tumor was calculated as: Volume = Length x (Width)² x 0.5 (cm³). As illustrated in Figure 4a, mAb 3.19.3 and Zactima™ significantly delayed the growth of the LoVo tumors as single agent. However the combination mAb 3.19.3 and ZD6474 had a significantly greater effect than the single agents alone as illustrated by the following values from tumour inhibition:
3.19.3 (10mg/kg 2xwk) = 48%; (p<0.001)
Zactima™ (50mg/kg/day) = 46%; (p<0.001)
Combination 3.19.3 + Zactima™ = 83% inhibition (p<0.001 for combination vs. single agent).

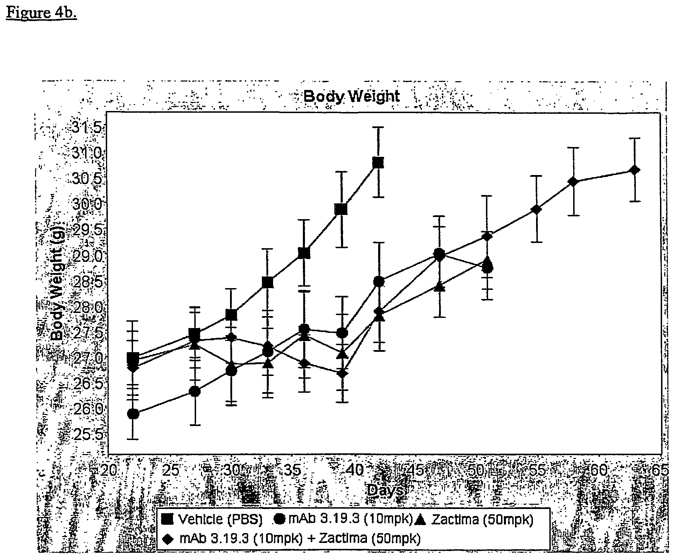

No additional toxicity was observed with the combinations as compared to single-agent treatment alone as determined by changes in body weights (Figure 4b). These results demonstrate that combination treatment with anti-Ang2 mAb 3.19.3 and the VEGF inhibitor Zactima™ leads to improvements in efficacy without additive toxicity in pre-clinical models and provide basis for further clinical investigation of this combination.

### Determination of the therapeutic efficacy of mAb 3.19.3 in combination with mAb DC101 in human SW620 colon tumour xenografts

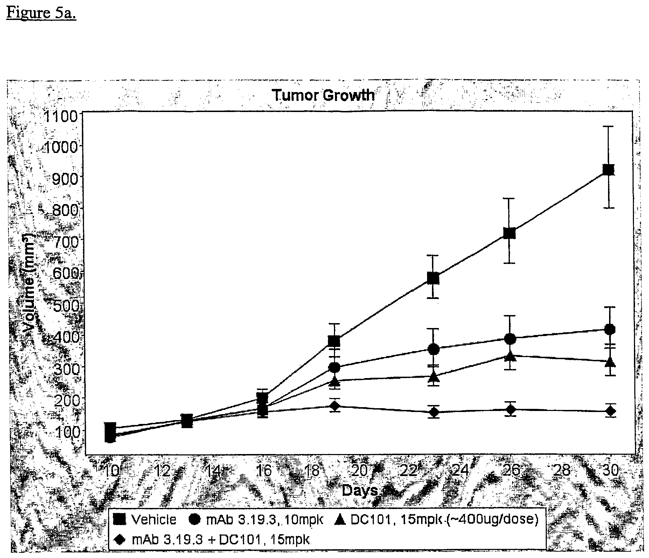

The anti-tumor activity of mAb 3.19.3 was evaluated in combination with monoclonal antiobody DC101 which is directed against VEGFR-2 /KDR, in the SW620 colorectal cancer xenograft model. Briefly, SW620 cells were cultured under routine tissue culture conditions in flasks until the cells reached sub-confluence. Immunodeficient 8-10 week old NCr nude mice were used, and cell suspensions containing approximately 1 x 106 cells were injected subcutaneously into the right flank of the mice. After the tumour volumes reached 100mm³, the mice were randomized in groups and the treatments were initiated. The mAb 3.19.3 10mg/kg in saline was injected intraperitoneally, twice per week for 3 weeks. The mAb DC101 15mg/kg in saline was also injected intraperitoneally, following the same schedule of twice per week for 3 weeks. The dimensions of each tumor were measured twice per week. The volume of the tumor was calculated as: Volume = Length x (Width)² x 0.5 (cm³). As illustrated in Figure 5a, the combination of mAb 3.19.3 and DC101 shows significantly greater activity than either single agent alone. This is also illustrated by the following values from tumour inhibition:
3.19.3 (10mg/kg 2xwk) = 48%; (p<0.03)
DC101 (15mg/kg 2xwk) = 66%; (p<0.01)
Combination 3.19.3 + DC101 = 93% inhibition (p<0.001 for combination vs. single agent).

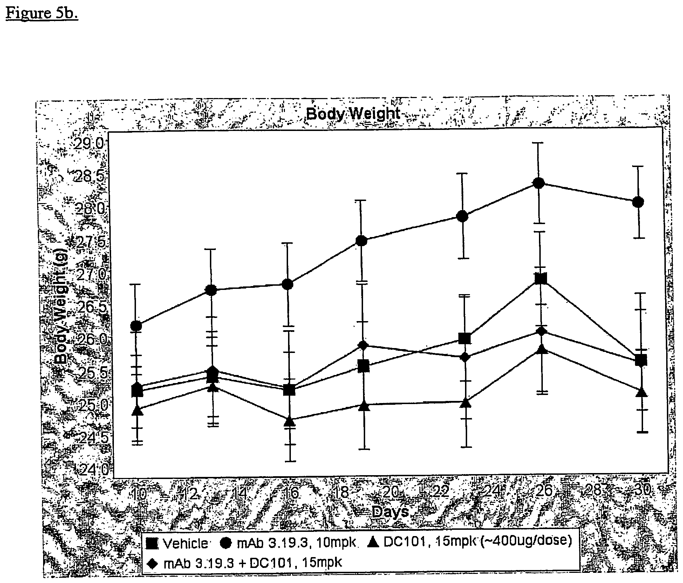

No additional toxicity was observed with the combinations as compared to single-agent treatment alone as determined by changes in body weights (Figure 5b). These results demonstrate that combination treatment with anti-Ang2 mAb 3.19.3 and the anti-VEGFR-2 antibody DC101 leads to significant improvements in efficacy without additive toxicity in pre-clinical models. This data provide basis for further clinical investigation of anti-Ang2 mAb 3.19.3 treatment together with other anti-angiogenic antibody combinations including AVASTIN™.

### Determination of the therapeutic efficacy of mAb 3.19.3 in combination with AVASTIN™ in human tumour xenografts

The anti-tumor activity of monoclonal antibody 3.19.3 in combination with AVASTIN™ can be evaluated in xenograft models of human tumors. A431, Colo205, LoVo or other cells can be cultured in flasks as routine until the cells reach sub-confluence. Immunodeficient 7-10 week old male or female NCR nude mice can be employed for model development. The cells can be harvested, suspended in Matrigel, and then injected subcutaneously into each mouse. The mice can then be randomized into cohorts containing 8-10 mice. AVASTIN™ and mAb 3.19.3 can be administered by intraperitoneal or intravenous injection. The dimensions of each tumour can be measured twice per week. The volume of the tumour can be calculated as: Volume = Length x (Width)² x 0.5 cm³, or by bilateral Vernier caliper measurement and, taking length to be the longest diameter across the tumor and width the corresponding perpendicular, calculated using the formula (π/6) x (length x width) x √ (length x width). Growth inhibition from the start of treatment can be assessed by comparison of the differences in tumor volume between control and treated groups.

The combination of mAb 3.19.3 in combination with AVASTIN™ treatment is expected to produce a significantly greater inhibition of tumour growth than either single agent alone (P<0.01 for single agent vs. combination: with P values derived by one-tailed two-sample *t*-test assuming equal variance).

### Determination of the therapeutic efficacy of mAb 3.19.3 in combination with SU11248 (Sutent) or BAY43-9006 (Sorafinib) in human tumour xenografts

The anti-tumor activity of monoclonal antibody 3.19.3 in combination with Sutent or Sorafinib can be evaluated in xenograft models of human tumors. HT29, A431, Colo205, LoVo or other human tumor cells can be cultured in flasks as routine until the cells reach sub-confluence. Immunodeficient 7-10 week old male or female NCR nude mice can be employed for model development. The cells can be harvested, suspended in Matrigel, and then injected subcutaneously into each mouse. The mice can then be randomized into cohorts containing 8-10 mice. Sutent and mAb 3.19.3 can be administered by intraperitoneal or intravenous injection according to the table below.

| **Group** | **Compound** | **Schedule** | **Dose (mg/kg)** | **# Animals** |
|---|---|---|---|---|
| 1 | Vehicle | b.i.d.x21 | | 10 |
| 2 | 3.19.3 | 2x/week for 3 weeks | 10 | 9 |
| 3 | Sutent | b.i.d.x21 | 40 | 9 |
| 4 | Sutent | b.i.d.x21 | 80 | 9 |
| 5 | Sutent 3.19.3 | b.i.d.x21 2x/week for 3 weeks | 40 | 9 |
| | | | 10 | |
| 6 | Sutent 3.19.3 | b.i.d.x21 2x/week for 3 weeks | 80 | 9 |
| | | | 10 | |

The dimensions of each tumour can be measured twice per week. The volume of the tumour can be calculated as: Volume = Length x (Width)² x 0.5 cm³, or by bilateral Vernier caliper measurement and, taking length to be the longest diameter across the tumor and width the corresponding perpendicular, calculated using the formula (π/6) x (length x width) x √ (length x width). Growth inhibition from the start of treatment can be assessed by comparison of the differences in tumor volume between control and treated groups.

The combination of mAb 3.19.3 in combination with Sutent or Sorafinib is expected to produce a significantly greater inhibition of tumour growth than either single agent alone (P<0.01 for single agent vs. combination: with P values derived by one-tailed two-sample *t-*test assuming equal variance).

The nucleotide and polypeptide sequences of the variable regions of the monoclonal antibodies as listed in Table 1 are shown below.

### Anti-Ang-2 Monoclonal Antibody 3.3.2

Nucleotide sequence of heavy chain variable region: 
GAGGTGCAGCTGGTGGAGTCTGGGGGAGGCTTGGTCCAGCCTGGGGGGTCCCTG
AGACTCTCCTGTGCAGTCTCTGGATTCACCTTTAGTAGCTATTGGATGAGCTGGGT
CCGCCAGGCTCCAGGGAAGGGGCTGGAGTGGGTGGCCAACATAAAGCAAGATGG
AAGTGAGAAATACTATGTGGACTCTGTGAAGGGCCGATTCACCATCTCCAGAGAC
AACGCCAAGAACTCACTGTATCTGCAAATGAACAGCCTGAGAGCCGAGGACACG
GCTGTGTATTACTGTGCGAGAGATCAAGGTATAGCAGTGGCTGGGCCCTTTGACT
ACTGGGGCCAGGGAACCCTGGTCACCGTCTCCTCAGCC (SEQ ID NO: 1)
Amino acid sequence of heavy chain variable region: 
EVQLVESGGGLVQPGGSLRLSCAVSGFTFSSYWMSWVRQAPGKGLEWVANIKQDGS
EKYYVDSVKGRFTISRDNAKNSLYLQMNSLRAEDTAVYYCARDQGIAVAGPFDYW
GQGTLVTVSSA (SEQ ID NO: 2)
Nucleotide sequence of light chain variable region: 
GAAATAGTGATGACGCAGTCTCCAGCCACCCTGTCTGTGTCTCCAGGGGAAAGAG
CCACCCTCTCCTGCAGGGCCAGTCAGACTGTTAGCAGCGACTTAGCCTGGTACCA
GCAGAAACCTGGCCAGGCTCCCAGGCTCCTCATCTATGGAGCATCCATTAGGGCC
ACTGGTATCCCAGCCAGGTTCAGTGGCAGTGGGTCTGGGACAGAGTTCACTCTCA
CCATCAGCAGCCTGCAGTCTGAAGATTTTGCAGTTTATTCCTGTCAGCAGTATTAT
AACTGGTGGACGTTCGGCCAAGGGACCAAGGTGGAAATCAAACGAA (SEQ ID
NO: 3)
Amino acid sequence of light chain variable region:

EIVMTQSPATLSVSPGERATLSCRASQTVSSDLAWYQQKPGQAPRLLIYGASIRATGIP
ARFSGSGSGTEFTLTISSLQSEDFAVYSCQQYYNWWTFGQGTKVEIKR (SEQ ID NO:
4)

### Anti-Angiopoietin-2 Monoclonal Antibody 3.19.3

Nucleotide sequence of heavy chain variable region: 
CAGGTGCAGCTGGTGGAGTCTGGGGGAGGCGTGGTCCAGCCTGGGAGGTCCCTG
AGACTCTCCTGTGCAGCCTCTGGATTCACCTTCACTAACTATGGCATGCACTGGG
GCCGCCAGGCTCCAGGCAAGGGGCTGGAGTGGGTGGCAGTTATATCACATGATG
GAAATAATAAGTATTATGTAGACTCCGTGAAGGGCCGATTCACCATCTCCAGAGA
CAATTCCAAGAACACGCTGTATCTGCAAATGAACAGCCTGAGAGCTGAGGACAC
GGCTGTGTATTACTGTGCGAGAGAGGGAATCGATTTTTGGAGTGGCCTCAACTGG
TTCGACCCCTGGGGCCAGGGAACCCTGGTCACCGTCTCCTCAGCC (SEQ ID NO: 5)
Amino acid sequence of heavy chain variable region: 
QVQLVESGGGVVQPGRSLRLSCAASGFTFTNYGMHWGRQAPGKGLEWVAVISHDG
NNKYYVDSVKGRFTISRDNSKNTLYLQMNSLRAEDTAVYYCAREGIDFWSGLNWFD
PWGQGTLVTVSSA (SEQ ID NO: 6)
Nucleotide sequence of light chain variable region: 
GAAATTGTGTTGACGCAGTCTCCAGGCACCCTGTCTTTGTCTCCAGGGGAAAGAG
CCACTCTCTCCTGCAGGGCCAGTCAGAGTATTACCGGCAGCTACTTAGCCTGGTA
CCAGCAGAAACCTGGCCAGGCTCCCAGACTCCTCATCTGTGGTGCATCCAGCTGG
GCCACTGGCATCCCAGACAGGTTCAGTGGCAGTGGGTCTGGGACAGACTTCACTC
TCACCATCAGTAGACTGGAGCCTGAAGATTTTGCAGTGTATTACTGTCAGCAGTA
TAGTAGTTCACCGATCACCTTCGGCCAAGGGACACGACTGGAGATTAAACGA
(SEQ ID NO: 7)
Amino acid sequence of light chain variable region:

EIVLTQSPGTLSLSPGERATLSCRASQSITGSYLAWYQQKPGQAPRLLICGASSWATGI
PDRFSGSGSGTDFTLTISRLEPEDFAVYYCQQYSSSPITFGQGTRLEIKR (SEQ ID NO:
8)

### Anti-Ang-2 Monoclonal Antibody 3.31.2

Nucleotide sequence of heavy chain variable region: 
GAGGTGCAGCTGGTGGAGTCTGGGGGAGGCTTGGTCCAGCCTGGGGGGTCCCTG
AGACTCTCCTGTGCAGCCTCTGGATTCACCTTTAGTAGCTATTGGATGAGCTGGGT
CCGCCAGGCTCCAGGGAAGGGGCTGGAGTGGGTGGCCAACATAAAGCAAGATGG
AAGTGACAAATACTATGTGGACTCTGTGAAGGGCCGATTCACCATCTCCAGAGAC
AACGCCAAGAACTCACTGTATCTGCGAATGAACAGCCTGAGAGCCGAGGACACG
 
GCTGTGTTTACTGTGCGAGAGATATGGGCAGTGGCTGGTTTGACTACTGGGGCC
AGGGAACCCTGGTCACCGTCTCCTCAGCC (SEQ ID NO: 9)
Amino acid sequence of heavy chain variable region: 
EVQLVESGGGLVQPGGSLRLSCAASGFTFSSYWMSWVRQAPGKGLEWVANIKQDGS
DKYYVDSVKGRFTISRDNAKNSLYLRMNSLRAEDTAVFYCARDMGSGWFDYWGQG
TLVTVSSA (SEQ ID NO: 10)
Nucleotide sequence of light chain variable region: 
GAAGTAGTGATGACGCAGTCTCCAGCCACCCTGTCTGTGTCTCCAGGGGAAAGAG
CCACCCTCTCCTGCAGGGCCAGTCAGAGTGTTGGCAGCAACTTAGCCTGGTACCA
GCAGAAACCTGGCCAGGCTCCCAGGCTCCTCATCTATGGTGCATCCACCAGGGCC
ACTGGTATCCCAGCCAGGTTCAGTGGCAGTGGGTCTGGGACAGAGTTCACTCTCA
CCATCAGCAGCCTGCAGTCTGAAGATTTTGCAGTTTATTGCTGTCAGCAGTATAAT
CACTGGTGGACGTTCGGCCAAGGGACCAAGGTGGAAATCAAACGA (SEQ ID NO:
11)
Amino acid sequence of light chain variable region:

EVVMTQSPATLSVSPGERATLSCRASQSVGSNLAWYQQKPGQAPRLLIYGASTRATG
IPARFSGSGSGTEFTLTISSLQSEDFAVYCCQQYNHWWTFGQGTKVEIKR (SEQ ID
NO: 12)

### Anti-Ang-2 Monoclonal Antibody 5.16.3

Nucleotide sequence of heavy chain variable region: 
CAGGTACAGCTGGTGCAGTCTGGGGCTGAGGTGAAGAAGCCTGGGGCCTCAGTG
AAGGTCTCCTGCAAGGCTTCTGGATACACCTTCACCGGCTTCTATATGTACTGGGT
GCGACAGGCCCCTGGACAAGGGCTTGAGTGGATGGGATGGATCAACCCTAACAG
TAGTGGCACAAACCATGCACAGAAGTTTCAGGGCAGGGTCACCATGACCAGGGA
CACGTCCATCAGCACAGCCTACATGGAGCTGAGCAGGCTGAGATCTGACGACAC
GGCCGTGTATTACTGTGCGAGAGATCAGGATATAGCAACAGCTGGTCCCTTTGAC
TACTGGGGCCAGGGAACCCTGGTCACCGTCTCCTCAGC (SEQ ID NO: 13)
Amino acid sequence of heavy chain variable region: 
QVQLVQSGAEVKKPGASVKVSCKASGYTFTGFYMYWVRQAPGQGLEWMGWINPN
SSGTNHAQKFQGRVTMTRDTSISTAYMELSRLRSDDTAVYYCARDQDIATAGPFDY
WGQGTLVTVSS (SEQ ID NO: 14)
Nucleotide sequence of light chain variable region: 
GAAATAGTGATGACGCAGTCTCCAGCCACCCTGTCTGTGTCTCCAGGGGAAAGAG
CCACCCTCTCCTGCAGGGCCAGTCAGAGTGTTAGCAGCAACTTAGCCTGGTACCA
GCAGAAACCTGGCCAGGCTCCCAGGCTCCTCATCTTTGGTGCATCCACCCGGGCC
ACTGGTATCCCAGCCAGGTTCAGTGGCAGTGGGTCTGGGACAGAGTTCACTCTCA
CCATCAGCAGCCTGCAGTCTGAAGATTTTGCAGTTTATTACTGTCAGCAGTATAAT
AACTGGTGGACGTTCGGCCGAGGGACCAAGGTGGAAATCAAACGAA (SEQ ID
NO: 15)
Amino acid sequence of light chain variable region:

EIVMTQSPATLSVSPGERATLSCRASQSVSSNLAWYQQKPGQAPRLLIFGASTRATGIP
ARFSGSGSGTEFTLTISSLQSEDFAVYYCQQYNNWWTFGRGTKVEIKR (SEQ ID NO:
16)

### Anti-Ang-2 Monoclonal Antibody 5.28.1

Nucleotide sequence of heavy chain variable region: 
GAAGTGCAGCTGGTGGAGTCTGGGGGAATCGTGGTACAGCCTGGGGGGTCCCTG
AGACTCTCCTGTGCAGCCTCTGGATTCACCTTTGATGATTATACCATGCACTGGGT
CCGTCAAACTCCGGGGAAGGGTCTGGAGTGGGTCTCTCTTATTAGTTGGGATGGT
GGTAGCACATACTATGCAGACTCTGTGAAGGGCCGATTCACCATCTCCAGAGACA
ACAGCAAAAACTCCCTGTATCTGCAAATGAACAGTCTGAGAACTGAGGACACCG
CCTTGTATTACTGTGCAAAAGATATAGATATAGCAGTGGCTGGTACAGGATTTGA
CCACTGGGGCCAGGGAACCCTGGTCACCGTCTCCTCAGCT (SEQ ID NO: 17)
Amino acid sequence of heavy chain variable region: 
EVQLVESGGIVVQPGGSLRLSCAASGFTFDDYTMHWVRQTPGKGLEWVSLISWDGG
STYYADSVKGRFTISRDNSKNSLYLQMNSLRTEDTALYYCAKDIDIAVAGTGFDHWG
QGTLVTVSSA (SEQ ID NO: 18)
Nucleotide sequence of light chain variable region: 
GAAATAGTGATGACGCAGTCTCCAGCCACCCTGTCTGTGTCTCCAGGGGAAAGAG
CCACCCTCTCCTGCAGGGCCAGTCAGAGTGTTACCAGCAACCTAGCCTGGTACCA
GCAGAAACCTGGCCAGGCTCCCAGGCTCCTCATCTATGGTGCATTAATTAGGGCC
ACTGGTATCCCAGCCAGGTTCAGTGGCAGTGGGTCTGGGACAGAGTTCACTCTCA
CCATCAGCAGCCTGCAGTCTGAAGATTTTGCAGTTTATTACTGTCAGCAATATAAT
 
AACTGGCCATTCACTTTCGGCCCTGGGACCAAAGTGGATATCAAACGA (SEQ ID
NO: 19)
Amino acid sequence of light chain variable region:

EIVMTQSPATLSVSPGERATLSCRASQSVTSNLAWYQQKPGQAPRLLIYGALIRATGIP
ARFSGSGSGTEFTLTISSLQSEDFAVYYCQQYNNWPFTFGPGTKVDIKR (SEQ ID NO:
20)

### Anti-Ang-2 Monoclonal Antibody 5.78.3

Nucleotide sequence of heavy chain variable region: 
CAGGTGCAGCTGGTGCAGTCTGGGGCTGAGGTGAAGAAGCCTGGGGCCTCAGTG
AAGGTCTCCTGCAAGGCTTCTGGATACACCTTCACCGGCTACTATATGCACTGGG
TGCGACAGGCCCCTGGACAAGGGCTTGAGTGGATGGGATGGATCAACCCTAACA
GTGGTGGCACAAACTATGCACAGAAGTTTCAGGGCAGGGTCACCATGACCAGGG
ACACGTCCATCAGCACAGCCTACATGGAGCTGAGCAGGCTGAGATCTGACGACA
CGGCCGTGTATTACTGTGCGAGAGATAGGGGCTGGAACTACGCAGACTACTACTA
CTACGGTATGGACGTCTGGGGCCAAGGGACCACGGTCACCGTCTCCTCAGCT
(SEQ ID NO: 21)
Amino acid sequence of heavy chain variable region: 
QVQLVQSGAEVKKPGASVKVSCKASGYTFTGYYMHWVRQAPGQGLEWMGWINPN
SGGTNYAQKFQGRVTMTRDTSISTAYMELSRLRSDDTAVYYCARDRGWNYADYYY
YGMDVWGQGTTVTVSSA (SEQ ID NO: 22)
Nucleotide sequence of light chain variable region: 
GACATCGTGATGACCCAGTCTCCAGACTCCCTGGCTGTGTCTCTGGGCGAGAGGG
CCACCATCAACTGCAAGTCCAGCCAGAGTGTTTTATACAGTTCCAACAATCAGAA
CTTCTTAGCTTGGTATCAGCAGAAACCAGGACAGCCTCCTAAACTGCTCATTTACT
GGGCATCTACCCGGGAATCCGGGGTCCCTGACCGATTCAGTGGCAGCGGGTCTGG
GACAGATTTCACTCTCACCATCAGCAGCCTGCAGGCTGAAGATGTGGCAGTTTAT
TACTGTCACCAATATTATAGTACTCCGATCACCTTCGGCCAAGGGACACGACTGG
AGATTAAACGA (SEQ ID NO: 23)
Amino acid sequence of light chain variable region:

DIVMTQSPDSLAVSLGERATINCKSSQSVLYSSNNQNFLAWYQQKPGQPPKLLIYWA
STRESGVPDRFSGSGSGTDFTLTISSLQAEDVAVYYCHQYYSTPITFGQGTRLEIKR
(SEQ ID NO: 24)

### Anti-Ang-2 Monoclonal Antibody 5.86.1

Nucleotide sequence of heavy chain variable region: 
CAGGTGCAGCTGGTGCAGTCCGGGGCTGAGGTGAAGAAGCCTGGGGCCTCAGTG
AAGGTCTCCTGCAAGGCTTCTGGATACACCTTCACCGGCTACCATATGTACTGGG
TGCGACAGGCCCCTGGACAAGGGCTTGAGTGGCTGGGATGGATCAACCCTAACA
GTGGTGGCACAAACTATGCACAGAAGTTTCAGGGCAGGGTCACCATGACCAGGG
ACACGTCCATCAGCACAGCCTACATGGAGCTGAGCAGGCTGAGATCTGACGACA
CGGCCGTGTATTACTGTGTGAGAGATCAGGGTATAGCAGCAGCTGGTCCCTTTGA
CTACTGGTGCCAGGGAACCCTGGTCACCGTCTCCTCAGCT (SEQ ID NO: 25)
Amino acid sequence of heavy chain variable region: 
QVQLVQSGAEVKKPGASVKVSCKASGYTFTGYHMYWVRQAPGQGLEWLGWINPNS
GGTNYAQKFQGRVTMTRDTSISTAYMELSRLRSDDTAVYYCVRDQGIAAAGPFDYW
CQGTLVTVSSA (SEQ ID NO: 26)
Nucleotide sequence of light chain variable region: 
GACATCCGGATGACCCAGTCTCCATCCTCCCTGTCTGCATCTGTTGGAGACAGAG
TCACCATCACTTGCCGGGCAAGTCAGCGCATTAGCACCTATTTAAATTGGTATCA
GCAGAAACCAGGGAAAGCCCCTAAGTTCCTGATCTATGCTGCATCTAGTTTGCAA
AGTGGGGTCCCATCAAGGTTCAGTGGCAGTGGATCTGGGACAGATTTCACTCTCA
CCATCAGCAGTCTGCAACCTGAAGATTTTGCAACTTACTACTGTCAACAGAGTTA
CACTACCCCATTCACTTTCGGCCCTGGGACCAAAGTGGATATCAAACGA (SEQ ID
NO: 27)
Amino acid sequence of light chain variable region:

DIRMTQSPSSLSASVGDRVTITCRASQRISTYLNWYQQKPGKAPKFLIYAASSLQSGVP
SRFSGSGSGTDFTLTISSLQPEDFATYYCQQSYTTPFTFGPGTKVDIKR (SEQ ID NO:
28)

### Anti-Ang-2 Monoclonal Antibody 5.88.3

Nucleotide sequence of heavy chain variable region: 
GAGGTGCAGATGGTGGAGTCTGGGGGAGGCTTGGTCCAGCCGGGGGGGTCCCTG
AGACTCTCCTGTGCAGCCTCTGGATTCACCTTAAGAAGCTACTGGATGAGCTGGG
TCCGCCAGGCTCCAGGGAAGGGGCTGGAGTGGGTGGCCAACATAAAGGAAGACG
GAAGTGAGAAATACCATGTGGACTCTGTGAAGGGCCGATTCACCATCTCCAGAG
ACAACGCCGAGAACTCACTGTTTCTGCAAATGAGCAGCCTGCGAGCCGAGGACA
CGGCTGTGTATTACTGTGCGAGAGATATGGAAGCATCAGCTGGCCTCTTTGACTA
CTGGGGCCAGGGAACCCTGGTCACCGTCTCCTCAGCT (SEQ ID NO: 29)
Amino acid sequence of heavy chain variable region: 
EVQMVESGGGLVQPGGSLRLSCAASGFTLRSYWMSWVRQAPGKGLEWVANIKEDG
SEKYHVDSVKGRFTISRDNAENSLFLQMSSLRAEDTAVYYCARDMEASAGLFDYWG
QGTLVTVSSA (SEQ ID NO: 30)
Nucleotide sequence of light chain variable region: 
GAAATAGTGATGACGCAGTCCCCAGCCACCCTGTCTGTGTCTCCAGGGGAAAGAG
CCATCCTCTCCTGCAGGGCCAGTCAGAGTATTAGCAGCAACTTAGCCTGGTACCA
GCAGAAACCTGGCCAGGCTCCCAGGCTCCTCATCTATGGTGCATCCACCAGGGCC
ACTGGTATCCCAGCCAGGTTCAGTGGCAGTGGGTCTGGGACAGAGTTCACTCTCA
CCATCAGCAGCCTGCAGTCTGAAGATTTTGCAGTTTATTACTGTCAGCAGTATAAT
TACTGGTGGACGTTCGGCCAAGGGACCAAGGTGGAAATCAAACGA (SEQ ID NO:
31)
Amino acid sequence of light chain variable region:

EIVMTQSPATLSVSPGERAILSCRASQSISSNLAWYQQKPGQAPRLLIYGASTRATGIP
ARFSGSGSGTEFTLTISSLQSEDFAVYYCQQYNYWWTFGQGTKVEIKR (SEQ ID NO:
32)

### Anti-Ang-2 Monoclonal Antibody 5.101.1

Nucleotide sequence of heavy chain variable region: 
CAGGTGCAGCTGGTGCAGTCTGGGGCTGAGGTGAAGAAGCCTGGGGCCTCAGTG
AAGGTCTCCTGCAAGGCTTCTGGATACACCTTCACCGGCTACTATATGCACTGGG
TGCCACAGGCCCCTGGACAAGGGCTTGAGTGGATGGGATGGATCAACCCTAACA
GTGGTGGCACAAACTATGCACAGAAGTTTCAGGGCAGGGTCACCATGACCAGGG
 
ACACGTCCATCAGCACAGCTTACATGGAGCTGAGGAGGCTGAGATCTGACGACA
CGGCCGTGTATTACTGTGCGAGAGATGGGGGTAGTATACCAGTGTCTGGTCACTT
TGACTACTGGGGGCAGGGAACCCTGGTCACCGTCTCCTCAGCT (SEQ ID NO: 33)
Amino acid sequence of heavy chain variable region: 
QVQLVQSGAEVKKPGASVKVSCKASGYTFTGYYMHWVPQAPGQGLEWMGWINPN
SGGTNYAQKFQGRVTMTRDTSISTAYMELRRLRSDDTAVYYCARDGGSIPVSGHFD
YWGQGTLVTVSSA (SEQ ID NO: 34)
Nucleotide sequence of light chain variable region: 
GAAATAGTGATGACGCAGTCTCCAGCCACCCTGTCTGTGTCTCCAGGGGAAAGAG
CCACCCTCTCCTGCAGGGCCAGTCAGAGTCTTATCAGCAACTTAGCCTGGTACCA
GCAGAAACCTGGCCAGGCTCCCAGGCTCCTCATCTTTGGTGCATCCACCAGGGCC
ACTGGTATCCCAGCCAGGTTCAGTGGCAGTGGGTCTGGGACAGAGTTCACTCTCA
CCATCAGCAGCCTGCAGTCTGAAGATTTTGCAGTTTATTACTGTCATCAGTATAAT
AACTGGTGGACGTTCGGCCAAGGGACCAAGGTGGAAATCAAACGA (SEQ ID NO:
35)
Amino acid sequence of light chain variable region:

EIVMTQSPATLSVSPGERATLSCRASQSLISNLAWYQQKPGQAPRLLIFGASTRATGIP
ARFSGSGSGTEFTLTISSLQSEDFAVYYCHQYNNWWTFGQGTKVEIKR (SEQ ID NO:
36)

### Anti-Ang-2 Monoclonal Antibody 5.103.1

Nucleotide sequence of heavy chain variable region: 
CAGGTGCAGCTGGTGCAGTCTGGGGCTGAGGTGAAAAAGCCTGGGGCCTCAGTC
AAGGTCTCCTGCAAGGCTTCTGGATACACCTTCACCGGCTACTATTTGTACTGGGT
GCCACAGGCCCCTGGACAAGGGCTTGAGTGGATGGGATGGATCAGCCCTAACAG
TGGTGGCACAAACTATGCACAGAAGTTTCAGGGCAGGGTCACCATGACCAGGGA
CACGTCCATCAGCACAGCCTACATGGAGCTGAGCAGGCTGAGATCTGACGACAC
GGCCGTGTATTACTGTGCGAGAGATCAGGTCATAGCAGTAGCTGGTCCCTTTGAC
TACTGGGCCCAAGGAACCCTGGTCACCGTCTCCTCAGCT (SEQ ID NO: 37)
Amino acid sequence of heavy chain variable region: 
QVQLVQSGAEVKKPGASVKVSCKASGYTFTGYYLYWVPQAPGQGLEWMGWISPNS
GGTNYAQKFQGRVTMTRDTSISTAYMELSRLRSDDTAVYYCARDQVIAVAGPFDYW
AQGTLVTVSSA (SEQ ID NO: 38)
Nucleotide sequence of light chain variable region: 
GAAACAGTGATGACGCAGTCTCCAGCCACCCTGTCTGTGTCTCCAGGGGAAAGAG
TCACCCTCTCCTGCAGGGCCAGTCAGAGTGTTATCAGCAGCTTAGCCTGGTACCA
GCAGAAACCTGGCCAGGCTCCCAGGCTCCTCATCTATGGTGCATCCACCAGGGCC
ACTGGTATCCCAGCCAGGTTCAGTGGCAGTGGGTCTGGGACAGAGTTCACTCTCA
CCATCAGCAGCCTGCAGTCTGAAGATTTTGCAGTTTATTACTGTCAGCAGTATAAT
AATTGGTGGACGTTCGGCCAAGGGACCAAGGTGGAAATCAAACGA (SEQ ID NO:
39)
Amino acid sequence of light chain variable region:

ETVMTQSPATLSVSPGERVTLSCRASQSVISSLAWYQQKPGQAPRLLIYGASTRATGIP
ARFSGSGSGTEFTLTISSLQSEDFAVYYCQQYNNWWTFGQGTKVEIKR (SEQ ID NO:
40)

All references cited herein, including patents, patent applications, papers, text books, and the like, and the references cited therein, to the extent that they are not already, are hereby incorporated herein by reference in their entirety.

The foregoing written specification is considered to be sufficient to enable one skilled in the art to practice the invention. The foregoing description and Examples detail certain preferred embodiments of the invention and describes the best mode contemplated by the inventors. It will be appreciated, however, that no matter how detailed the foregoing may appear in text, the invention may be practiced in many ways and the invention should be construed in accordance with the appended claims and any equivalents thereof.

## Claims

1. A combination for use in treating disease related angiogenesis, wherein the combination comprises:
(i) an anti-Angoipoietin-2 (Ang-2) antagonist antibody that inhibits binding of Ang-2 to Tie-2 receptor; and
(ii) an anti- VEGF antibody that inhibits binding of VEGF-A, to one or more of the receptors selected from the group consisting of VEGFR1 and VEGFR2.

2. The combination of claim 1, wherein the anti-Ang-2 antagonist antibody is a monoclonal antibody comprising:
(i) a heavy chain variable region comprising SEQ ID NO: 6; and
(ii) a light chain variable region comprising SEQ ID NO: 8.

3. The combination of claim 1, wherein the anti-Ang-2 antagonist antibody is a monoclonal antibody that binds to the same epitope as an antibody comprising:
(i) a heavy chain variable region comprising SEQ ID NO: 6; and
(ii) a light chain variable region comprising SEQ ID NO: 8.

4. The combination of any one of claims 1 to 3, wherein the anti-Ang-2 antagonist antibody is a monoclonal antibody having at least 95% identity to an antibody comprising:
(i) a heavy chain variable region comprising SEQ ID NO: 6; and
(ii) a light chain variable region comprising SEQ ID NO: 8.

5. The combination of any one of claims 1 to 4, wherein the anti-VEGF antibody is selected from: DC101, Avastin, CDP791 and IMC1121b.

6. The combination of any one of claims 1 to 5, wherein VEGF-A is selected from VEGF-121, VEGF-145, VEGF-165, VEGF-183, VEGF-189, and VEGF-206.

7. The combination of claim 6, wherein VEGF-A is VEGF-165.

8. The combination of any one of claims 1 to 7, wherein disease related angiogenesis is related to non-solid tumors and solid tumors.

9. The combination of claim 8, wherein the non-solid tumors may be selected from leukaemia, multiple myeloma, and haematologic malignancies, including lymphoma.

10. The combination of claim 8, wherein the solid tumors may be selected from solid tumors and their metastases, such as, melanoma, non-small cell lung cancer, glioma, hepatocallular carcinoma, glioblastoma, and carcinoma, such as, thyroid, bile duct, bone, gastric, CNS, including brain, head and neck, hepatic, stomach, prostate, breast, renal, testicular, ovarian, skin, cervical, lung, muscle, neuronal, oesophageal, bladder, lung, uterine, vulval, endometrial, kidney, colorectal, pancreatic, membranes such as pleural and peritoneal, salivary gland, and epidermoid.

11. The combination of claim 8, wherein the disease related angiogenesis is related to colorectal, lung, kidney tumors or brain carcinoma.

12. The combination of claim 8, wherein the disease related angiogenesis is related to haematologic malignancies, and breast, prostate, head and neck, gastric and liver carcinoma.

13. The combination of any one of claims 1 to 12, further comprising anti-proliferative drugs, anti-neoplastic drugs, or a combination thereof.

14. The combination of claim 13, wherein the anti-proliferative or anti-neoplastic drugs are selected from the group consisting of:
(i) alkylating agents, for example, cis-platin, carboplatin, oxaliplatin, cyclophosphamide, nitrogen mustard, melphalan, chlorambucil, busulphan and nitrosoureas;
(ii) anti-metabolites, for example, antifolates, such as, fluoropyrimidines like 5-fluorouracil and tegafur, raltitrexed, gemcitabine, capecitabine, methotrexate, pemetrexed, cytosine arabinoside and hydroxyurea, (2)-2-{o-fluoro-p-[N-{2, 7- dimethyl-4-oxo-3,4-dihydroquinazolin-6-ylmethyi)-N(prop-2-ynyl)amino]benzamido}-4-(tetrazol-5-yl)butyric acid);
(iii) combinations which comprise an alkylating agent and an antimetabolite, for example Folfox;
(iv) anti-tumour antibiotics, for example, anthracyclines like adriamycin, bleomycin, doxorubicin, daunomycin, epirubicin, idarubicin, mitomycin-C, dactinomycin and mithramycin;
(v) anti-mitotic agents, for example, vinca alkaloids like vincristine, vinblastine, vindesine and vinorelbine and taxoids like taxol and taxotere; and
(vi) topoisomerase inhibitors, for example, epipodophyllotoxins like etoposideand teniposide, irinotecan, amsacrine, topotecan and camptothecin.
